# EUROPEAN PATENT APPLICATION

(11) **EP 4 279 510 A1**
(43) Date of publication of application: **22.11.2023**
(21) Application number: 22739667.8
(22) Date of filing: 12.01.2022
(51) Int. Cl.: C07K 16/28, A61P 35/00, G01N 33/574, G01N 33/563, A61K 39/00

(54) **NOVEL ANTIBODY TO CD55 AND USES THEREOF**

(30) Priority: 12.01.2021 KR 20210004196; 05.01.2022 KR 20220001356
(71) Applicant: SG Medical Inc., Songpa-gu Seoul 05548 (KR); Korea University Research and Business Foundation, Seoul 02741 (KR); Korea Atomic Energy Research Institute, Daejeon 34057 (KR)
(72) Inventor: LEE, Ji Chul, Gwangmyeong-si Gyeonggi-do 14244 (KR); PARK, Hye In, Seoul 05328 (KR); MIN, Sung-Won, Seoul 04701 (KR); KWON, Hyeong Sun, Seoul 02253 (KR); JUNG, Sang Taek, Seoul 06217 (KR); LIM, Jae Cheong, Sejong 30150 (KR); DOH, So Hee, Seoul 06344 (KR); CHO, Eun Ha, Sejong 30062 (KR); LEE, So-Young, Daejeon 34090 (KR); JUNG, Sung Hee, Gongju-si Chungcheongnam-do 32625 (KR)
(74) Representative: V.O.
(86) International application number: PCT/KR2022/000551
(87) International publication number: WO 2022/154472

(57) **Abstract**

The present disclosure relates to an antibody binding specifically to CD55 or an antigen-binding fragment thereof; and a composition for preventing, treating and/or diagnosing cancer containing the same. The antibody of the present disclosure may be used as an effective therapeutic composition for various CD55-mediated diseases since it shows high binding ability and inhibitory effect for the CD55 protein which promotes tumor growth by inhibiting the complement immune mechanism. In addition, the antibody of the present disclosure may be usefully used as an effective therapeutic adjuvant that fundamentally removes drug resistance and remarkably improves therapeutic responsiveness in various diseases in which resistance to therapeutic agents with CDC (complement-dependent cytotoxicity) as a mechanism of action has been induced due to overexpression of CD55.

## Description

### [Technical Field]

The present disclosure relates to a novel antibody which specifically recognizes CD55 or an antigen-binding fragment thereof and a composition for preventing, treating and/or diagnosing cancer using the same.

### [Background Art]

The global anticancer drug market has grown at an annual average rate 10-13% in the past decade, and is expected to reach a total of 200 billion dollars in 2022. As of 2020, one out of five men and one out of six women has experienced cancer at least once in lifetime globally, and one out of eight men and one out of eleven women died of cancer. The prevalence rate and mortality rate of cancer are increasing continuously.

The world anticancer drug market is shifting in paradigm from the 1st generation chemotherapy to the 2nd generation targeted anticancer therapy and the 3rd generation anticancer immunotherapy. Beyond the early therapeutic method aiming at simply reducing or inhibiting cancer, targeted anticancer therapy of selectively attacking cancer cells only without harming normally dividing cells is being researched actively. The development of targeted anticancer therapy consists two steps of the screening of receptors that are expressed specifically in cancer cells and the development of targeting compounds that bind to the receptors.

CD55 (decay-accelerating factor, DAF) is a receptor that inhibits the complement immune mechanism. It is highly expressed in various solid cancers such as colorectal cancer, lung cancer, stomach cancer, breast cancer, ovarian cancer, leukemia, head and neck cancer, etc. and promotes the proliferation of cancer cells by inhibiting the complement immune system from killing cancer cells. Accordingly, CD55 has been actively researched as a target molecule in targeted anticancer therapy.

Especially, since solid cancers where CD55 is expressed highly show low therapeutic responsiveness to various anticancer drugs having complement-dependent cytotoxicity (CDC) as the mechanism of action, the development of an excellent antibody therapeutic agent capable of binding to CD55 and effectively inhibiting its activity with higher affinity and specificity is necessary.

A number of papers and patent documents are referenced throughout the present specification and their citations are indicated. The disclosures of the cited papers and patent documents are incorporated by reference into the present specification as a whole, so that the level of the technical field to which the present disclosure belongs and the content of the present disclosure are more clearly described.

### [References of Related Art]

### [Patent Documents]

Patent document 1. Korean Patent Registration No. 10-1800774.

### [Disclosure]

### [Technical Problem]

The inventors of the present disclosure have specifically identified the CD55 protein, which is a receptor that inhibits the complement immune mechanism, and have made consistent efforts to develop a superior CD55 inhibitor capable of effectively repairing the immune mechanism inhibited by CD55 by specifically inhibiting its activity. As a result, they have found out that the activity of complementmediated cell lysis for cancer cells, etc. is recovered significantly when an antibody with some amino acids in the key antigen recognition site of a variable region substituted is used due to significantly increased CD55-binding ability and inhibitory effect, and have completed the present disclosure.

The present disclosure is directed to providing an antibody binding specifically to CD55 or an antigen-binding fragment thereof; and a composition for preventing, treating and/or diagnosing cancer, which contains the same.

The present disclosure is also directed to providing a bispecific antibody including an antigen-binding fragment of an antibody binding specifically to CD55 and an antigen-binding fragment of an antibody binding specifically to CD20.

Other purposes and advantages of the present disclosure will become clearer by the following detailed description, claims and drawings.

### [Technical Solution]

In an aspect, the present disclosure provides an antibody binding specifically to CD55 or an antigen-binding fragment thereof, which includes a heavy chain variable region including an HCDR1 region having an amino acid sequence represented by the general formula 1; an HCDR2 region having an amino acid sequence of SEQ ID NO 1; and an HCDR3 region having an amino acid sequence represented by the general formula 2:

**General formula 1** D-R-G-M-X₁ ;

**General formula 2** N-A-X₂-A-G-G-W-H-A-A-Y-I-D-A,

wherein X₁ is Ala or Val, and X₂ is selected from a group consisting of Val, Ala, Ile, Leu, Phe and Met.

The inventors of the present disclosure have specifically identified the CD55 protein, which is a receptor that inhibits the complement immune mechanism, and have made consistent efforts to develop a superior CD55 inhibitor capable of effectively repairing the immune mechanism inhibited by CD55 by specifically inhibiting its activity. As a result, they have found out that the activity of complementmediated cell lysis for cancer cells, etc. is recovered significantly when an antibody with some amino acids in the key antigen recognition site of a variable region substituted is used due to significantly increased CD55-binding ability and inhibitory effect.

In the present specification, the term "antibody" refers to a protein molecule which includes an immunoglobulin molecule having immunological reactivity with a specific antigen and acts as a receptor that specifically recognizes the antigen, and includes both a polyclonal antibody and a monoclonal antibody.

The antibody includes not only a complete full-length antibody but also an antigen-binding fragment (antibody fragment) of the antibody molecule. A complete antibody has two full-length light chains and two full-length heavy chains. The light chains are connected to the heavy chains by disulfide bonds. The heavy chain constant region has gamma (γ), mu (µ), alpha (α), delta (δ) and epsilon (ε) types and has gamma 1 (γ₁), gamma 2 (γ₂), gamma 3 (γ₃), gamma 4 (γ₄), alpha 1 (α₁) and alpha 2 (α₂) subclasses. The light chain constant region has kappa (κ) and lambda (λ) types.

In the present specification, the term "antigen-binding fragment of an antibody" refers to a fragment of a whole antibody molecule which has the ability of specifically recognizing an antigen and binding to the antigen, and includes a single-domain antibody (sdAb), a single-chain antibody (scFv), Fab, F(ab'), F(ab')₂, Fv, etc. Examples of the fragment include a monovalent fragment consisting of V_{L}, V_{H}, C_{L} and C_{H}1 domains (Fab fragment); a divalent fragment including two Fab fragments linked by a disulfide bridge at the hinge region (F(ab')₂ fragment); an Fd fragment consisting of V_{H} and C_{H}1 domains; an Fv fragment consisting of the V_{L} and V_{H} domains of one arm of an antibody and a disulfide-linked (sdFv); a dAb fragment consisting of a V_{H} domain; and a separated complementarity-determining region (CDR) or a combination of two or more separated CDRs optionally linked by a inker. In addition, the V_{L} and V_{H} domains of the scFv may be linked by a linker to be paired and form a single protein chain with a monovalent molecular structure. Such as single-chain antibody is also included in the antibody fragment. Furthermore, the antibody or antibody fragment includes a tetrameric antibody including two heavy chain molecules and two light chain molecules; an antibody light chain monomer; an antibody heavy chain monomer; an antibody light chain dimer; an antibody heavy chain dimer; an intrabody; a monovalent antibody; a camel antibody; and a single-domain antibody (sdAb).

Fv is a minimum antibody fragment having only a heavy chain variable region and a light chain variable region. Recombinant techniques for producing Fv fragments are disclosed in International PCT Publication Nos. WO 88/10649, WO 88/106630, WO 88/07085, WO 88/07086 and WO 88/09344. A variable region of a heavy chain and a variable region of a light chain are noncovalently linked in the two-chain Fv, whereas a variable region of a heavy chain and a variable region of a light chain are generally covalently linked by a peptide linker in the single-chain Fv or a dimer-like structure such as the two-chain Fv can be formed through linkage directly to the C-terminal. Such an antibody fragment can be obtained using a protease (For example, a whole antibody may be cleaved with papain to obtain Fab or may be cleaved with pepsin to obtain an F(ab')₂ fragment.) or prepared through gene recombinant technology.

In the present specification, the term "heavy chain" includes both a full-length heavy chain and a fragment thereof having a variable region sequence sufficient to confer specificity for an antigen, including a variable region domain V_{H} and three constant region domains C_{H}1, C_{H}2 and C_{H}3. And, in the present specification, the term "light chain" includes both a full-length heavy light and a fragment thereof having a variable region sequence sufficient to confer specificity for an antigen, including a variable region domain V_{L} and a constant region domain C_{L}.

In the present specification, the term "CDR (complementarity-determining region)" refers to the amino acid sequence of a hypervariable region of a heavy chain and a light chain of an immunoglobulin (Kabat et al. Sequences of Proteins of Immunological Interest, 4th Ed., U.S. Department of Health and Human Services, National Institutes of Health (1987)). The heavy chain (HCDR11, HCDR2 and HCDR3) and the light chain (LCDR1, LCDR2 and LCDR3) includes three CDRs. The CDRs provide major contact residues for binding of the antibody to an antigen or an epitope.

The antibody or antibody fragment of the present disclosure includes variants having conservative amino acid substitutions in the CDR region, and may include the variants for the amino acid sequences described in the attached sequence list within the range that allows specific recognition of CD55. For example, additional changes may be made to the amino acid sequence of the antibody in order to improve half-life, biocompatibility or other biological properties other than binding affinity. When considering variations having such biologically equivalent activity, it should be construed that the antibody of the present disclosure or a nucleic acid molecule encoding the same also includes sequences exhibiting substantial identity to the listed sequences. The substantial identity refers to an identity of at least 61%, specifically at least 70%, more specifically at least 80%, further more specifically at least 90%, exhibited when the sequence of the present disclosure is aligned to match as much as possible with any other sequence. Methods of the alignment for comparison of sequences are known in the art. Various methods and algorithms for the alignment are disclosed in Smith and Waterman, Adv. Appl. Math. (1981) 2: 482; Needleman and Wunsch, J. Mol. Bio. (1970) 48: 443; Pearson and Lipman, Methods in Mol. Biol. (1988) 24: 307-31; Higgins and Sharp, Gene (1988) 73: 237-44; Higgins and Sharp, CABIOS (1989) 5: 151-3; Corpet et al. Nuc. Acids Res. (1988) 16: 10881-90; Huang et al. Comp. Appl. BioSci. (1992) 8: 155-65; and Pearson et al. Meth. Mol. Biol. (1994) 24: 307-31.

In the present specification, the term "affinity" refers to the strength of binding between an antibody or an antigen-binding fragment and an antigen, and may also be expressed as "binding ability".

In a specific exemplary embodiment of the present disclosure, X₁ in the general formula 1 is Ala.

In a specific exemplary embodiment of the present disclosure, X₂ in the general formula 2 is Val.

More specifically, the heavy chain variable region of the present disclosure includes one or more framework region (FR) selected from a group consisting of HFR1 to HFR4:
HFR1: EVQLY₁ESGGGLVQPGGSLRLSCY₂AY₃GFTFS (Y₁ is Val or Ala, Y₂ is Val or Ala, and Y₃ is Ser or Arg),
HFR2: WVRQY₄PGKGLEWVS (Y₄ is Ala or Ser),
HFR3: RY₅TISRDNSKNTLYLQMNY₆LRAEDTAVYYCAK (Y₅ is Ala or Thr, and Y₆ is Ser or Asn), and
HFR4: WGQGTTVTVSS.

Further more specifically, the heavy chain variable region of the present disclosure has an amino acid sequence selected from a group consisting of SEQ ID NOS 19-22.

In a specific exemplary embodiment of the present disclosure, the antibody or antigen-binding fragment thereof further includes a light chain variable region including an LCDR1 region having an amino acid sequence of SEQ ID NO 2; LCDR2 region having an amino acid sequence represented by the general formula 3; and an LCDR3 region having an amino acid sequence of SEQ ID NO 3:

**General formula 3** W-N-X₃-K-R-P-S

wherein X₃ is Asn or Asp.

More specifically, X₃ is Asp.

More specifically, the light chain variable region of the present disclosure includes one or more framework region (FR) selected from a group consisting of LFR1 to LFR4:
LFR1: SYELTQPPSVSVSPGQTASITC,
LFR2: WY₇QQKPGQSPVTVIY (Y₇ is Phe or Tyr) (F or Y),
HFR3: GIPERFSGSKSGNTATLTISGTQAMDEADYYC, and
HFR4: FGGGTKLTVL.

Further more specifically, the light chain variable region of the present disclosure has an amino acid sequence of SEQ ID NO 4 or 18.

In another aspect, the present disclosure provides an antibody binding specifically to CD55 or an antigen-binding fragment thereof, which includes:
a) a light chain variable region of SEQ ID NO 4; or a light chain variable region including one or more substitution selected from a group consisting of substitution of Y (Tyr) of the 32nd amino acid sequence of the light chain variable region of SEQ ID NO 4 with F (Phe) and substitution of D (Asp) of the 48th amino acid sequence with N (Asn), and
b) a heavy chain variable region of SEQ ID NO 5; or a heavy chain variable region including one or more substitution selected from a group consisting of substitution of V (Val) of the 5th amino acid sequence in the heavy chain variable region of SEQ ID NO 5 with A (Ala), substitution of A (Ala) of the 23rd amino acid sequence with V (Val), substitution of S (Ser) of the 25th amino acid sequence with R (Arg), substitution of A (Ala) of the 35th amino acid sequence with V (Val), substitution of A (Ala) of the 40th amino acid sequence with S (Ser), substitution of A (Ala) of the 68th amino acid sequence with T (Thr), substitution of S (Ser) of the 85th amino acid sequence with N (Asn) and substitution of G (Gly) of the 101st amino acid sequence with V (Val), L (Leu), I (Ile), F (Phe), M (Met) or A (Ala).

In another aspect, the present disclosure provides a nucleic acid molecule encoding the antibody or antigen-binding fragment thereof of the present disclosure.

In the present specification, the term "nucleic acid molecule" comprehensively includes DNA (gDNA and cDNA) and RNA molecules. A nucleotide which is the basic building block of the nucleic acid molecule include not only a natural nucleotide but also an analogue with the sugar or base moiety modified (Scheit, Nucleotide Analogs, John Wiley, New York (1980); Uhlman and Peyman, Chemical Reviews, (1990) 90: 543-584). The sequence of the nucleic acid molecule encoding the heavy chain or light chain variable region of the present disclosure may be varied. The variation includes addition, deletion, nonconservative substitution or conservative substitution of a nucleotide.

The nucleic acid molecule of the present disclosure is interpreted to include a nucleotide sequence that exhibits substantial identity to the nucleotide sequence. The substantial identity refers to an identity of at least 80%, specifically at least 90%, more specifically at least 95%, exhibited when the sequence of the present disclosure is aligned to match as much as possible with any other sequence.

In another aspect, the present disclosure provides a composition for preventing or treating cancer, which contains the antibody or antigen-binding fragment thereof of the present disclosure as an active ingredient.

In another aspect, the present disclosure provides a method for preventing or treating cancer, which includes a step of administering a pharmaceutically effective amount of the antibody or antigen-binding fragment thereof of the present disclosure to a subject.

In another aspect, the present disclosure provides the antibody or antigen-binding fragment thereof of the present disclosure for use in therapy.

In the present specification, the term "prevention" refers to prevention of a disease or a disorder in a subject who has not been diagnosed with the disease or disorder but has the risk of having the disease or disorder.

In the present specification, the term "treatment" refers to (a) suppression of the development of a disease, a disorder or symptoms; (b) alleviation of a disease, a disorder or symptoms; or (c) removal of a disease, a disorder or symptoms. When the composition of the present disclosure is administered to a subject, it suppresses the development of cancer by recovering the complement immune mechanism and inhibiting the proliferation of cancer cells by inhibiting the activity of the CD55 receptor, or removes or alleviates symptoms thereof. Accordingly, the composition of the present disclosure may be used as a composition for treating cancer on its own, or may be administered in combination with another pharmaceutical ingredient, e.g., a therapeutic agent having CDC (complement-dependent cytotoxicity) as a mechanism of action, as a therapeutic adjuvant for improving its therapeutic responsiveness. Therefore, in the present specification, the term "treatment" or "therapeutic agent" encompasses the meaning of "aid of treatment" or "therapeutic adjuvant".

In the present specification, the term "administration" or "administer" refers to direct administration of a therapeutically effective amount of the composition of the present disclosure to a subject so that the same amount can be formed in the body of the subject.

In the present specification, the term "therapeutically effective amount" refers to an amount of the pharmaceutical composition of the present disclosure wherein the content of a pharmaceutical ingredient contained in the composition is enough to provide therapeutic or prophylactic effect in a subject and, therefore, encompasses the meaning of "prophylactically effective amount".

In the present specification, the term "subject" includes human, mouse, rat, guinea pig, dog, cat, horse, cow, pig, monkey, chimpanzee, baboon or rhesus monkey, without limitation. Specifically, the subject of the present disclosure is human.

In a specific exemplary embodiment of the present disclosure, the composition further contains one or more pharmaceutical ingredient selected from a group consisting of an anti-CD20 antibody and a tyrosine kinase inhibitor.

More specifically, the anti-CD20 antibody is rituximab.

More specifically, the tyrosine kinase inhibitor is selected from a group consisting of imatinib and gefitinib.

In a specific exemplary embodiment of the present disclosure, the cancer that can be prevented or treated with the composition of the present disclosure is CD55-positive cancer.

In the present specification, the term "CD55-positive cancer" refers to a carcinoma of cancer cells that express the CD55 receptor, specifically cancer in which CD55 is expressed such that the complement immune activity in a subject is inhibited measurably. Therefore, the term "CD55-positive cancer" encompasses "CD55-overexpressed cancer". According to the present disclosure, the composition of the present disclosure can remove drug resistance and enhance treatment sensitivity in various cancers wherein resistance to therapeutic agents has been induced due to the overexpression of CD55.

The CD55-positive cancer includes, for example, melanoma, lymphoma, lung cancer, thyroid cancer, breast cancer, colorectal cancer, prostate cancer, head and neck cancer, stomach cancer, liver cancer, bladder cancer, kidney cancer, cervical cancer, pancreatic cancer, leukemia, bone marrow cancer, ovarian cancer, sarcoma, bile duct cancer and endometrial cancer. But, it includes all solid cancers and blood cancers expressing CD55, without being limited thereto.

In another aspect, the present disclosure provides a composition for diagnosing cancer, which contains the antibody or antigen-binding fragment thereof of the present disclosure as an active ingredient.

The description of the antibody or the antigen-binding fragment used in the present disclosure and the cancer to which it is applied will be omitted to avoid unnecessary redundancy.

In the present specification, the term "diagnosis" includes determination of a subject's susceptibility to a particular disease, determination of whether a subject currently has a particular disease, and determination of the prognosis of a subject suffering from a particular disease.

The CD55 receptor is a typical cancer diagnostic marker that shows a higher expression level in cancer tissues than in normal tissues. The antibody of the present disclosure that binds specifically to CD55 can accurately detect the presence of a tumor in a biological sample. In addition, the antibody of the present disclosure can be usefully used to establish an early customized therapeutic strategy depending on the nature of cancer by accurately measuring the expression level of CD55 in a tumor through various immunoassay methods and predicting the treatment sensitivity of the tumor to a CDC drug.

In the present specification, the term "composition for diagnosis" refers to an integrated mixture or device including the antibody of the present disclosure as a means of measuring the expression level of the CD55 protein to determine whether a subject has cancer or to predict treatment sensitivity to a CDC drug and, therefore, may also be expressed as a "diagnostic kit".

In the present specification, the term "biological sample" includes a tissue, a cell, whole blood, serum, plasma, saliva, urine, lymph, spinal fluid, a tissue autopsy sample (brain, skin, lymph node, spinal cord, etc.), a cell culture supernatant, a disrupted eukaryotic cell and a bacterial expression system, although not being limited thereto.

The detection of the CD55 protein in the biological sample can be performed through detection of antigen-antibody complex formation by a colorimetric method, an electrochemical method, a fluorimetric method, a luminometric method, a particle counting method, visual assessment or a scintillation counting method. In the present specification, the term "detection" refers to a series of processes for determining whether an antigen-antibody complex has been formed. The detection can be carried out using various markers including enzymes, fluorophores, ligands, luminophores, microparticles or radioisotopes.

The enzymes used as detection markers include, for example, acetylcholinesterase, alkaline phosphatase, β-D-galactosidase, horseradish peroxidase and β-latamase, the fluorophores include fluorescein, Eu³⁺, Eu³⁺ chelate or cryptate, etc., the ligands include biotin derivatives, etc., the luminophores include acridinium esters, isoluminol derivatives, etc., the microparticles include colloidal gold, colored latex, etc., and the radioisotopes include ⁵⁷Co, ³H, ¹²⁵I, ¹²⁵I-Bonton Hunter reagent, etc.

In an exemplary embodiment of the present disclosure, the antigen-antibody complex may be detected by enzyme-linked immunosorbent assay (ELISA). The antibody of the present disclosure may have a detection label. When it does not have a detection label, it may be detected by treating with another antibody which can capture the antibody of the present disclosure and has a detection label.

In another aspect, the present disclosure provides a bispecific antibody or a functional fragment thereof including the antigen-binding fragment of the antibody binding specifically to CD20 of the present disclosure.

In the present specification, the term "bispecific antibody" refers to an antibody including two different antigen-binding regions defined by different amino acid sequences. Typically, the two antigen-binding regions included in the bispecific antibody are specific for different antigens or epitopes.

The bispecific antibody of the present disclosure is an antibody molecule (CD20 x CD55) wherein the antigen-binding fragment specific for CD55 of the present disclosure and another antigen-binding fragment specifically recognizing CD20 are bound. According to the present disclosure, co-administration of an anti-CD55 antibody (e.g., EP-10H) and an anti-CD20 antibody (e.g., rituximab) exhibits better therapeutic effect for rituximab-resistant tumors.

In a specific exemplary embodiment of the present disclosure, the antigen-binding fragment of the anti-CD55 antibody included in the bispecific antibody is scFv.

In a specific exemplary embodiment of the present disclosure, the antigen-binding fragment of the antibody binding specifically to CD55 includes scFv.

In a specific exemplary embodiment of the present disclosure, the antigen-binding fragment of the antibody binding specifically to CD55 is V_{L}-linker-V_{H}-C_{H}2-C_{H}3.

In a specific exemplary embodiment of the present disclosure, the antigen-binding fragment of the anti-CD20 antibody included in the bispecific antibody is Fab.

In a specific exemplary embodiment of the present disclosure, the antigen-binding fragment of the antibody binding specifically to CD20 includes Fab.

In a specific exemplary embodiment of the present disclosure, the antigen-binding fragment of the antibody binding specifically to CD20 is V_{L}-C_{K}-linker-V_{H}-C_{H}1-C_{H}2-C_{H}3.

In a specific exemplary embodiment of the present disclosure, the antibody binding specifically to CD20 is rituximab.

The bispecific antibody of the present disclosure may include an Fc region consisting of first and second subunits that can associate stably. In the present specification, the term "Fc region" refers to the C-terminal region of an antibody heavy chain that includes at least a portion of the constant region of a full-length antibody, and includes both a wild-type Fc region and a variant Fc region. The Fc region of IgG includes IgG C_{H}2 and IgG C_{H}3 domains. The C_{H}3 domain of the bispecific antibody of the present disclosure may be a wild-type or variant C_{H}3 domain (e.g., a C_{H}3 domain having a "protruding part" ("knob") introduced in one chain thereof and a "cavity" ("hole") introduced in another chain thereof).

In a specific exemplary embodiment of the present disclosure, the bispecific antibody of the present disclosure may use a knobs-into-holes to prevent unwanted pairing of the heavy chains of CD20 x CD55. In the "knobs-into-holes" method [US 5,731,168; US7,695,936; Ridgway et al., Prot Eng 9, 617-621 (1996); Carter, J Immunol Meth 248, 7-15 (2001)], a protruding part ("knob") is introduced at the interface of a first polypeptide and a cavity ("hole") corresponding thereto is introduced at the interface of a second polypeptide in order to facilitate the formation of heterodimers and inhibit the formation of homodimers. The protruding part is formed by replacing a small amino acid residue at the interface of the first polypeptide with a larger one (e.g., tyrosine or tryptophan). The complementary cavity with the same or similar size as the protruding part is formed at the interface of the second polypeptide by replacing a large amino acid residue with a smaller one (e.g., alanine or threonine).

In a specific exemplary embodiment of the present disclosure, the bispecific antibody of the present disclosure may be one wherein an antigen-binding fragment of an anti-CD55 antibody in the form of V_{L}-linker-V_{H}-C_{H}2-C_{H}3 and an antigen-binding fragment of an anti-CD20 antibody in the form of V_{L}-C_{K}-linker-V_{H}-C_{H}1-C_{H}2-C_{H}3 are bound.

In a specific exemplary embodiment of the present disclosure, the bispecific antibody of the present disclosure may use a linker consisting of 10-100, more specifically 15-60, of Gly and Ser in order to prevent unwanted pairing of the heavy chains of CD20 x CD55.

Specifically, the bispecific antibody of the present disclosure may be one wherein CD55-scFv-knob (V_{L}-linker20-V_{H}-C_{H}2-C_{H}3) and CD20-Fab-hole (V_{L}-C_{K}-linker40-V_{H}-C_{H}1-C_{H}2-C_{H}3) are bound.

The linker20 refers to a linker including 20 Gly's or Ser's and may be, for example, Gly Gly Gly Gly Ser Gly Gly Gly Gly Ser Gly Gly Gly Gly Ser Gly Ser Gly Gly Ser, although not being limited thereto.

The linker40 refers to a linker including 20 Gly's or Ser's and may be, for example, Gly Gly Gly Gly Ser Gly Gly Gly Gly Ser Gly Gly Gly Gly Ser Gly Ser Gly Gly Ser Gly Gly Gly Gly Ser Gly Gly Gly Gly Ser Gly Gly Gly Gly Ser Gly Ser Gly Ser Ser, although not being limited thereto.

### [Advantageous Effects]

The features and advantages of the present disclosure may be summarized as follows:
(a) The present disclosure provides an antibody binding specifically to CD55 or an antigen-binding fragment thereof; and a composition for preventing, treating and/or diagnosing cancer, which contains the same.
(b) The antibody of the present disclosure may be used as an effective therapeutic composition for various CD55-mediated diseases since it shows high binding ability and inhibitory effect for the CD55 protein which promotes tumor growth by inhibiting the complement immune mechanism.
(c) In addition, the antibody of the present disclosure may be usefully used as an effective therapeutic adjuvant that fundamentally removes drug resistance and remarkably improves therapeutic responsiveness in various diseases in which resistance to therapeutic agents with CDC (complement-dependent cytotoxicity) as a mechanism of action has been induced due to overexpression of CD55.

### [Brief Description of Drawings]

FIG. 1 shows an ELISA analysis result of measuring the affinity of 4-1H and G4-1H for CD55.
FIG. 2 shows an ELISA analysis result of measuring the affinity of antibodies screened from a library wherein random mutation has been introduced to the G4-1H variable region for CD55.
FIG. 3 shows an ELISA analysis result of measuring the affinity of antibodies obtained by substituting the key site for improving binding ability with hydrophobic amino acids for CD55.
FIG. 4 shows a FACS assay result of analyzing the effect of co-treatment of rituximab and EP-10H on the death of BJAB cells.
FIG. 5a shows a FACS assay result of comparatively analyzing the expression level of CD20 and CD55 in Ramos and Ramos-RR cells. FIG. 5b shows a FACS assay result of analyzing the effect of co-treatment of rituximab and EP-10H antibody on the death of Ramos-RR cells.
FIG. 6a shows a FACS assay result of comparatively analyzing the expression level of CD55 in K562 and K562-IR cells. FIG. 6b shows a FACS assay result of analyzing the effect of co-treatment of imatinib and EP-10H antibody on the death of 562-IR cells.
FIG. 7a shows a FACS assay result of comparatively analyzing the expression level of CD55 in PC9 and PC9-GR cells. FIG. 7b shows a FACS assay result of analyzing the effect of co-treatment of imatinib and EP-10H antibody on the death of PC9-GR cells.
FIG. 8 schematically compares a CD20 x CD55 bispecific antibody of the present disclosure (SBU) with a bispecific antibody reported by Macor.
FIG. 9 shows an SDS-PAGE analysis result for a CD20 x CD55 bispecific antibody of the present disclosure (SBU).
FIGS. 10a and 10b show an ELISA analysis result of measuring the affinity of a CD20 x CD55 bispecific antibody for CD20 (FIG. 10a) and CD55 (FIG. 10b).
FIG. 11a shows a FACS assay result of analyzing the effect of CD20 x CD55 bispecific antibodies on the death of BJAB cells. FIG. 11b shows a result of comparing the death rate of BJAB cells treated with rituximab alone, co-treated with rituximab and EP-10H antibody, and treated with CD20 x CD55 bispecific antibodies.
FIG. 12a shows a FACS assay result of analyzing the effect of CD20 x CD55 bispecific antibodies on the death of Ramos cells. FIG. 12b shows a result of comparing the death rate of Ramos cells treated with rituximab alone, co-treated with rituximab and EP-10H antibody, and treated with CD20 x CD55 bispecific antibodies.
FIG. 13a shows a FACS assay result of analyzing the effect of CD20 x CD55 bispecific antibodies on the death of Ramos-RR cells. FIG. 13b shows a result of comparing the death rate of Ramos-RR cells treated with rituximab alone, co-treated with rituximab and EP-10H antibody, and treated with CD20 x CD55 bispecific antibodies.
FIG. 14 shows a result of investigating C4d production in supernatants of Ramos-RR cells treated with rituximab and CD20 x CD55 bispecific antibody (SBU).

### [Best Mode]

Hereinafter, the present disclosure will be described in more detail through examples. The examples are only for explaining the present disclosure more specifically and it will be obvious to those having ordinary knowledge in the art that that the scope of the present disclosure is not limited by them.

### Examples

### Example 1: Humanization of 4-1H

Humanization was performed as follows to reduce the immunogenicity of 4-1H disclosed in Korean Patent Application No. 10-2016-0132333.

For humanization of 4-1H, three FRs (LFR1, LFR2 and LFR3) in the light chain variable region of 4-1H were replaced with the FRs of IGLV3-1*01, and three FRs (HFR1, HFR2 and HFR3) in the heavy chain variable region were replaced with the FRs of IGHV3-23D*02. Then, specific amino acids were replaced with the amino acids of the parent antibody (4-1H) again in consideration of the similarity or dissimilarity of physical and chemical properties. The amino acid residues replaced again with the amino acids of the parent antibody (4-1H) were L46T and N66K in the light chain variable region and F68A in the heavy chain variable region. The amino acid residues in the antibody domain were designated according to the Kabat EU numbering system commonly used in the art (Kabat et al., "Sequences of Proteins of Immunological Interest", 5th Ed., U.S. Department of Health and Human Services, NIH Publication No. 91-3242, 1991). The J gene of the light chain variable region was fixed as FGGGTKLTVL with reference to US 2010-0056386, and the J gene of the heavy chain variable region was fixed as WGQGTTVTVSS with reference to US 2014-0206849. The humanized 4-1H antibody was named G4-1H and its base sequence is shown in Table 1.

**[Table 1]**

| Light chain variable region | | |
|---|---|---|
| G4-1H (V_{L}) | SYELTQPPSVSVSPGQTASITCSGGGGSYGWYQQKPGQSPV TVIYWNDKRPSGIPERFSGSKSGNTATLTISGTQAMDEADYYC GGWDSSTYAIFGGGTKLTVL | SEQ ID NO 4 |

| Heavy chain variable region | | |
|---|---|---|
| G4-1H (V_{H}) | EVQLVESGGGLVQPGGSLRLSCAASGFTFSDRGMAWVRQAP GKGLEWVSGISSSGRYTYYAPAVKGRATISRDNSKNTLYLQM NSLRAEDTAVYYCAKNAGAGGWHAAYIDAWGQGTTVTVSS | SEQ ID NO 5 |

| | | |
|---|---|---|
| Heavy chain and light chain variable region amino acid sequences of G4-1H | | |

In the above table, the underline indicates the CDR regions and the bold type indicates the amino acids replaced with those of the parent antibody (4-1H).

### Example 2: Complete antibody conversion and expression/purification of G4-1H clone

The DNA of the variable region of the G4-1H antibody constructed in Example 1 was synthesized as scFv (Cosmogenetech, Korea) and then converted to a complete antibody (full-length IgG) through PCR. First, the fragments of heavy chain and light chain variable regions and constant regions were obtained from a pUC vector (Cosmogenetech, Korea) including scFv through PCR using a combination of V_{H}, C_{H} and V_{L}, C_{K} primers described in Table 2. The heavy chain and light chain of G4-1H were obtained by conducting PCR using the obtained variable region and constant region of the antibody and a combination of HC and LC primers described in Table 2. The heavy chain was treated with EcoRI and *Not*I enzymes (New England Biolabs, UK) and then ligated into a pCMV vector (ThermoFisher Scientific, USA), which is an animal cell expression vector, treated with the same restriction enzymes. The light chain was also treated with *Xba*I (New England Biolabs, UK) and then ligated into a pCMV vector treated with the restriction enzyme. The ligated plasmid was transformed into DH5α competent cells (New England Biolabs, UK) by applying heat shock and the plasmid was obtained by culturing the resulting colony in large scale.

**[Table 2]**

| Primers | | Sequences | SEQ ID NO |
|---|---|---|---|
| V_{H} | Forward | CTT CCT GTC AGT AAC TAC AGG TGT CCA CTC CCA GGT GCA ACT GCA GCA GTC | 6 |
| | Reverse | CCA GCG TGA CCG TAT CCA GCG CCT CCA CCA AGG GCC CCA | 7 |
| C_{H} | Forward | GCC TCC ACC AAG GGC CC | 8 |
| | Reverse | TCC CCC GGC AAG TGA GCG GCC GCT CAC | 9 |
| HC | Forward | CAG AAT TCA CTC TAA CCA TGG AAT GGA GCT GGG TCT TTC TCT TCT TCC TGT CAG TAA CTA CAG | 10 |
| | Reverse | TCC CCC GGC AAG TGA GCG GCC GCT CAC | 9 |
| V_{L} | Forward | GGT CTT TGT ATA CAT GTT GCT GTG GTT GTC TGG TGT TGA AGG AAG CTA CGA GCT GAC AC | 11 |
| | Reverse | CAA GCT CAC CGT CTT GCG GAC CGT GGC C | 12 |
| C_{K} | Forward | CGG ACC GTG GCC GCC CCC TC | 13 |
| | Reverse | CGG GGC GAG TGC TAG TTC TAG AAC TA | 14 |
| LC | Forward | GGG AAT TCT AGA GGA TCG AAC CCT TTG CAA GCT TCG GCA CGA GCA GAC CAG CAT GGG CAT CAA GAT GGA GAC ACA TTC TCA GGT CTT TGT ATA CAT GTT G | 15 |
| | Reverse | CGG GGC GAG TGC TAG TTC TAG AAC TA | 14 |

| | | | |
|---|---|---|---|
| Primers used for complete antibody cloning of G4-1H | | | |

The plasmids of the heavy chain and the light chain were transduced into Expi293F cells (Invitrogen, USA) using polyethyleneimine (PEI) (Polysciences, USA) and 150 mM NaCl, and the cells were suspension-cultured for 7 days in a Freestyle 293 expression medium (Invitrogen, USA) in an Erlenmeyer flask under the condition of 37 °C, 8% CO₂ and 55% humidity. After centrifuging the cell culture at 4,000 rpm for 10 minutes, the supernatant was filtered with a 0.22-µm filter. The filtered supernatant was induced to bind to 1 mL of MabSelect SuRe resin (GE Healthcare, USA) at 4 °C. The resin was washed with a 10 cv (column volume) of 20 mM disodium phosphate and 500 mM sodium chloride (pH 7.0) solutions. After eluting the bound antibody using a 100 mM citric acid (pH 3.0) solution, it was neutralized with 1 M Tris-HCl (pH 9.0). After exchanging buffer with pH 7.2-7.4 PBS using a Slide-A-Lyzer dialysis cassette (ThermoFisher Scientific, USA), the size and purity of the light chain and heavy chain of the purified antibody were identified finally by SDS-PAGE. As a result, a molecular weight consistent with the theoretical value and high purity were confirmed.

### Example 3: Analysis of affinity of 4-1H and G4-1H for CD55

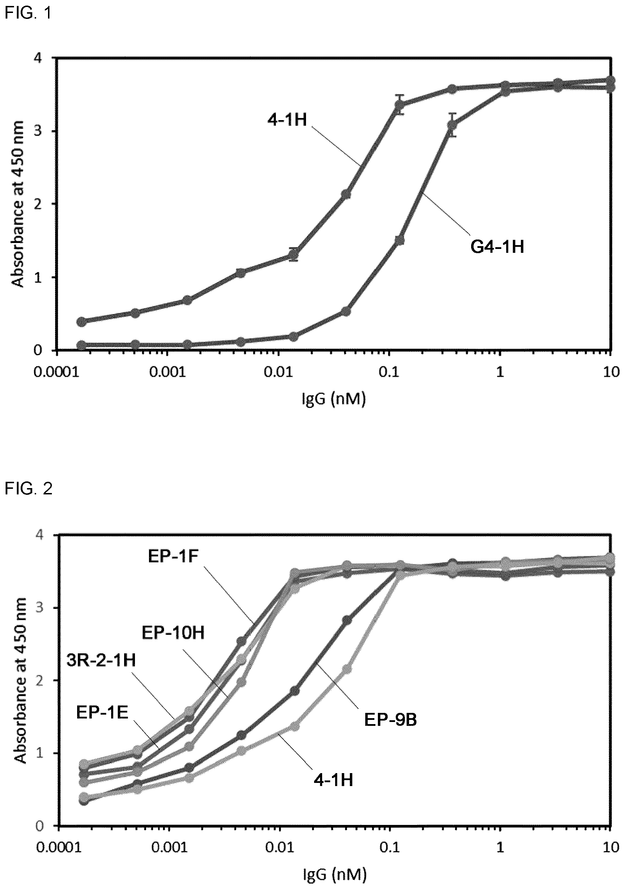

The binding ability of the G4-1H monoclonal antibody prepared in Example 2 for CD55 was investigated by indirect ELISA. For the indirect ELISA, recombinant human CD55 (R&D Systems, USA) diluted to 1 µg/mL in 50 µL of PBS was added to a 96-well immunoplate (Corning, USA) and then adsorbed by keeping at 4 °C overnight. After incubation for 1 hour at room temperature in a buffer containing 3% bovine serum albumin (Millipore, USA), followed by washing with a buffer containing 0.5% Tween 20 (Amresco, USA) three times, 50 µL of the serially diluted antibody (0.0001, 0.0003, 0.001, 0.003, 0.01, 0.03, 0.1, 0.3, 1, 3 and 10 nM) was treated per well. After incubation at room temperature for 2 hours for antibody-antigen binding, the antibody-antigen complex was washed three times with a buffer containing 0.5% Tween 20 (Amresco, USA). After treating with 50 µL of anti-human immunoglobulin Fc-HRP antibody (Jackson Immunoresearch, USA) diluted to 1:3,000 per well, followed by incubation at room temperature for 1 hour and washing three times with a buffer containing 0.5% Tween 20 (Amresco, USA), color development was conducted for 10 minutes by adding 50 µL of 3,3',5,5'-tetramethylbenzidine (TMB) (ThermoFisher Scientific, USA). As a result of measuring absorbance at 450 nm using a spectrophotometer (Biotek, USA), G4-1H showed lower affinity for CD55 than 4-1H (FIG. 1).

### Example 4: Construction of library with random mutation introduced in variable region

In order to improve the affinity of G4-1H for CD55, a library wherein random mutation is induced in the variable region of the G4-1H antibody was constructed through error-prone PCR. Specifically, after adding scFv sequencing primer 1 and primer 2, a proofreading-deficient polymerase (Takara, Japan), a polymerase buffer, dATP, dTTP, dCTP and dGTP to a pComb3x vector including G4-1H scFv, the degree of mutation was controlled by adjusting salt concentration and the amount of manganese ions. Error-prone PCR was performed by repeating 30 cycles of preheating at 94 °C for 5 minutes, separation of double helices at 91 °C for 1 minute, primer binding at 55 °C for 1 minute and DNA synthesis at 72 °C for 3 minutes, followed by DNA synthesis at 72 °C for 5 minutes. The purified PCR product and a pComb3x vector, which is a phagemid vector, were cleaved using a Sfil restriction enzyme (New England Biolabs, UK), ligated, and then transformed into ER2738 *E*. *coli.*

**[Table 3]**

| scFv sequencing primers | | |
|---|---|---|
| 1 | ACA CTT TAT GCT TCC GGC | SEQ ID NO 16 |
| 2 | CAA AAT CAC CGG AAC CAG AG | SEQ ID NO 17 |

| | | |
|---|---|---|
| Primers used in error-prone PCR | | |

### Example 5: Screening of clones with improved affinity for CD55 as compared to 4-1H

Clones with improved affinity for CD55 as compared to G4-1H and 4-1H were screened through biopanning using the library constructed in Example 4.

First, 2.5 µg of recombinant human CD55 (R&D Systems 2009-CD/CF, USA) was conjugated to magnetic beads (Invitrogen, USA). After binding the phage having affinity for CD55 by incubating the phage library and the CD55 conjugated to the magnetic beads at room temperature for 2 hours, the product was incubated with 4-1H antibody at room temperature for 30 minutes for competition. After washing with a buffer containing 0.5% Tween 20 (Amresco, USA) and eluting using an acidic solution, the eluted phage was infected into *E*. *coli* ER2738 for the next round of panning and then proliferated by culturing overnight. This procedure was repeated four times. The number of washing was increased from once in the first panning, 3 times in the second and third panning and 5 times in the fourth panning to obtain phages with high binding ability.

192 clones screened from the products of the third and fourth biopanning were incubated overnight at 37 °C on a 96-deep well plate after adding 100 µg/mL carbenicillin, 70 µg/mL kanamycin and VCSM13 helper phage to induce the proliferation of phages wherein the antibody was expressed. After centrifuging the culture, the supernatant containing the phage was incubated at 37 °C for 2 hours on an ELISA plate coated with recombinant human CD55 (R&D Systems 2009-CD/CF, USA). Then, the antibody binding to CD55 was identified by ELISA using HRP-conjugated anti-M13 antibody (Merck, USA) as a secondary antibody. The phage of a 4-1H clone was used as a control group, and five clones exhibiting higher binding ability were screened.

### Example 6: Complete antibody conversion and expression/purification of screened clones

Cloning was conducted to convert EP-1E, EP-1F, EP-9B, 3R-2-1H and EP-10H to full-length IgGs. The CDR sequences of EP-1E, EP-1F, EP-9B, 3R-2-1H and EP-10H are described in Table 4.

**[Table 4]**

| | Amino acid sequences | SEQ ID NO |
|---|---|---|
| EP-1E light chain variable region | SYELTQPPSVSVSPGQTASITCSGGGGSYGWFQQKPGQ SPVTVIYWNNKRPSGIPERFSGSKSGNTATLTISGTQAMD EADYYCGGWDSSTYAIFGGGTKLTVL | 18 |
| EP-1E heavy chain variable region | EVQLVESGGGLVQPGGSLRLSCAASGFTFSDRGMAWV RQAPGKGLEWVSGISSSGRYTYYAPAVKGRATISRDNSK NTLYLQMNSLRAEDTAVYYCAKNAVAGGWHAAYIDAWG QGTTVTVSS | 19 |
| EP-1F light chain variable region | SYELTQPPSVSVSPGQTASITCSGGGGSYGWYQQKPGQ SPVTVIYWNDKRPSGIPERFSGSKSGNTATLTISGTQAMD EADYYCGGWDSSTYAIFGGGTKLTVL | 4 |
| EP-1F heavy chain variable region | EVQLAESGGGLVQPGGSLRLSCAASGFTFSDRGMAWV RQAPGKGLEWVSGISSSGRYTYYAPAVKGRATISRDNSK NTLYLQMNSLRAEDTAVYYCAKNAVAGGWHAAYIDAWG | 20 |
| | QGTTVTVSS | |
| EP-9B light chain variable region | SYELTQPPSVSVSPGQTASITCSGGGGSYGWYQQKPGQ SPVTVIYWNDKRPSGIPERFSGSKSGNTATLTISGTQAMD EADYYCGGWDSSTYAIFGGGTKLTVL | 4 |
| EP-9B heavy chain variable region | EVQLVESGGGLVQPGGSLRLSCAASGFTFSDRGMVWV RQSPGKGLEWVSGISSSGRYTYYAPAVKGRATISRDNSK NTLYLQMNNLRAEDTAVYYCAKNAVAGGWHAAYIDAWG QGTTVTVSS | 21 |
| 3R-2-1H light chain variable region | SYELTQPPSVSVSPGQTASITCSGGGGSYGWYQQKPGQ SPVTVIYWNDKRPSGIPERFSGSKSGNTATLTISGTQAMD EADYYCGGWDSSTYAIFGGGTKLTVL | 4 |
| 3R-2-1H heavy chain variable region | EVQLVESGGGLVQPGGSLRLSCVARGFTFSDRGMAWV RQAPGKGLEWVSGISSSGRYTYYAPAVKGRTTISRDNSK NTLYLQMNSLRAEDTAVYYCAKNAVAGGWHAAYIDAWG QGTTVTVSS | 22 |
| EP-10H light chain variable region | SYELTQPPSVSVSPGQTASITCSGGGGSYGWYQQKPGQ SPVTVIYWNDKRPSGIPERFSGSKSGNTATLTISGTQAMD EADYYCGGWDSSTYAIFGGGTKLTVL | 4 |
| EP-10H heavy chain variable region | EVQLVESGGGLVQPGGSLRLSCAASGFTFSDRGMAWV RQAPGKGLEWVSGISSSGRYTYYAPAVKGRATISRDNSK NTLYLQMNSLRAEDTAVYYCAKNAVAGGWHAAYIDAWG QGTTVTVSS | 19 |

| | | |
|---|---|---|
| Amino acid sequences of light chain and heavy chain variable regions of screened clones | | |

In the above table, the underline indicates the CDR regions and the bold type indicates amino acids different from those of G4-1H. Because the antibodies screened in Example 5, EP-1E, EP-1F, EP-9B, 3R-2-1H and EP-10H, are in the form of scFv, they were converted to complete antibodies. The complete antibody conversion and expression/purification were conducted in the same manner as in Example 2.

**[Table 5]**

| Primers | | Sequences | SEQ ID NO |
|---|---|---|---|
| VL | Forward | GGT CTT TGT ATA CAT GTT GCT GTG GTT GTC TGG TGT TGA AGG AAG CTA CGA GCT GAC AC | 11 |
| | Reverse | CAA GCT CAC CGT CTT GCG GAC CGT GGC C | 12 |
| C_{K} | Forward | CGG ACC GTG GCC GCC CCC TC | 13 |
| | Reverse | CGG GGC GAG TGC TAG TTC TAG AAC TA | 14 |
| LC | Forward | GGG AAT TCT AGA GGA TCG AAC CCT TTG CAA GCT TCG GCA CGA GCA GAC CAG CAT GGG CAT CAA GAT GGA GAC ACA TTC TCA GGT CTT TGT ATA CAT GTT G | 15 |
| | Reverse | CGG GGC GAG TGC TAG TTC TAG AAC TA | 14 |
| V_{H} | Forward | CTT CCT GTC AGT AAC TAC AGG TGT CCA CTC CGA GGT CCA GCT GGT C | 23 |
| | Reverse | GTG ACC GTG TCC AGC GCC TCC ACC AAG GG | 24 |
| C_{H} | Forward | GCC TCC ACC AAG GGC CC | 8 |
| | Reverse | TCC CCC GGC AAG TGA GCG GCC GCT CAC | 9 |
| HC | Forward | CAG AAT TCA CTC TAA CCA TGG AAT GGA GCT GGG TCT TTC TCT TCT TCC TGT CAG TAA CTA CAG | 10 |
| | Reverse | TCC CCC GGC AAG TGA GCG GCC GCT CAC | 9 |

| | | | |
|---|---|---|---|
| Primers used in complete antibody cloning of selected clones | | | |

### Example 7: Analysis of affinity of screened complete antibodies for CD55

The affinity of the antibodies screened in Example 6 for CD55 was investigated by indirect ELISA. The indirect ELISA was conducted in the same manner as in Example 3.

As a result of ELISA, the antibody of Example 6 exhibited higher affinity for CD55 than 4-1H (FIG. 2). EP-1E, EP-1F, 3R-2-1H and EP-10H showed comparable affinity, and EP-9B exhibited affinity higher than that of 4-1H but lower than those of other antibodies. The antibodies exhibiting higher affinity for CD55 than 4-1H can be very usefully used for preparation of a pharmaceutical composition for diagnosing or treating various cancers.

### Example 8: Substitution of amino acids at key site for improving binding ability

In all the clones described in Table 4, G at the H100A site of G4-1H according to the Kabat EU numbering system was replaced with V. This site is included in the HCDR3 region, which is important for antibody-antigen binding. The inventors of the present disclosure identified this site as a key site for improving binding ability, and performed cloning to replace the site with hydrophobic amino acids other than V. 10H(A), 10H(I), 10H(L), 10H(F) and 10H(M) prepared using EP-10H, which is different from G4-1H only at the H100A site, as a basic backbone have the following CDR sequences (Table 6).

**[Table 6]**

| | Amino acids | SEQ ID NO |
|---|---|---|
| 10H(A) heavy chain variable region | EVQLVESGGGLVQPGGSLRLSCAASGFTFSDRGMA WVRQAPGKGLEWVSGISSSGRYTYYAPAVKGRATIS RDNSKNTLYLQMNSLRAEDTAVYYCAKNAAAGGWH AAYIDAWGQGTTVTVSS | 25 |
| 10H(I) heavy chain variable region | EVQLVESGGGLVQPGGSLRLSCAASGFTFSDRGMA WVRQAPGKGLEWVSGISSSGRYTYYAPAVKGRATIS RDNSKNTLYLQMNSLRAEDTAVYYCAKNAIAGGWHA AYIDAWGQGTTVTVSS | 26 |
| 10H(L) heavy chain variable region | EVQLAESGGGLVQPGGSLRLSCAASGFTFSDRGMA WVRQAPGKGLEWVSGISSSGRYTYYAPAVKGRATIS RDNSKNTLYLQMNSLRAEDTAVYYCAKNALAGGWH AAYIDAWGQGTTVTVSS | 27 |
| 10H(F) heavy chain variable region | EVQLAESGGGLVQPGGSLRLSCAASGFTFSDRGMA WVRQAPGKGLEWVSGISSSGRYTYYAPAVKGRATIS RDNSKNTLYLQMNSLRAEDTAVYYCAKNAFAGGWH AAYIDAWGQGTTVTVSS | 28 |
| 10H(M) heavy chain variable region | EVQLAESGGGLVQPGGSLRLSCAASGFTFSDRGMA WVRQAPGKGLEWVSGISSSGRYTYYAPAVKGRATIS RDNSKNTLYLQMNSLRAEDTAVYYCAKNAMAGGWH | 29 |
| | AAYIDAWGQGTTVTVSS | |

In the heavy chain variable region of EP-10H, which is a complete IgG cloned into a pCMV vector (ThermoFisher Scientific, USA), an animal cell expression vector, the amino acid in the complementarity-determining region of the heavy chain with mutation other than G4-1H was substituted with a hydrophobic amino acid.

First, the heavy chain variable region and constant region were obtained from the pCMV vector including the heavy chain variable region of EP-10H through PCR using combinations of HC1 and HC2 primers described in Table 4. The heavy chain variable region and constant region obtained through the PCR were subjected to overlap PCR using the HC3 forward primer and the HC2 reverse primer to obtain V_{H}-C_{H}1-C_{H}2-C_{H}3. The obtained heavy chain was cleaved using EcoRI and *Not*I restriction enzymes (New England Biolabs, UK) and ligated to a pCMV vector (ThermoFisher Scientific, USA), which is an animal cell expression vector treated with the same restriction enzymes. The ligated plasmid was transformed into DH5α *E*. *coli* (New England Biolabs, UK) by applying heat shock and the plasmid was obtained by culturing in large scale.

**[Table 7]**

| Primers | | Sequences | SEQ ID NO |
|---|---|---|---|
| HC1 | Forward | CTT CCT GTC AGT AAC TAC AGG TGT CCA CTC CGA GGT CCA GCT GGT C | 23 |
| | Reverse | GTA CTA CTG CGC CAA G | 30 |
| HC2 | Forward 1 | GTA CTA CTG CGC CAA GAA CGC CGC GGC CGG GGG GTG GCA CGC | 31 |
| | Forward 2 | GTA CTA CTG CGC CAA GAA CGC CAT TGC CGG GGG GTG GCA CGC | 32 |
| | Forward 3 | GTA CTA CTG CGC CAA GAA CGC CCT GGC CGG GGG GTG GCA CGC | 33 |
| | Forward 4 | GTA CTA CTG CGC CAA GAA CGC CAT GGC CGG GGG GTG GCA CGC | 34 |
| | Forward 5 | GTA CTA CTG CGC CAA GAA CGC CTT TGC CGG GGG GTG GCA CGC | 35 |
| | Reverse | TCC CCC GGC AAG TGA GCG GCC GCT CAC | 9 |
| HC3 | Forward | CAG AAT TCA CTC TAA CCA TGG AAT GGA GCT GGG TCT TTC TCT TCT TCC TGT CAG TAA CTA CAG | 10 |
| | Reverse | TCC CCC GGC AAG TGA GCG GCC GCT CAC | 9 |

| | | | |
|---|---|---|---|
| Primers used for amino acid substitution of key site for improving binding ability | | | |

After the cloning was completed, the complete antibodies were expressed and purified in the same manner as in Example 2 using the EP-10H light chain plasmid and the heavy chain plasmid of Table 6.

### Example 9: Analysis of affinity of complete antibodies prepared by substitution at key site for improving binding ability for CD55

The affinity of the antibodies prepared in Example 8 for CD55 was investigated by indirect ELISA. The indirect ELISA was conducted in the same manner as in Example 3.

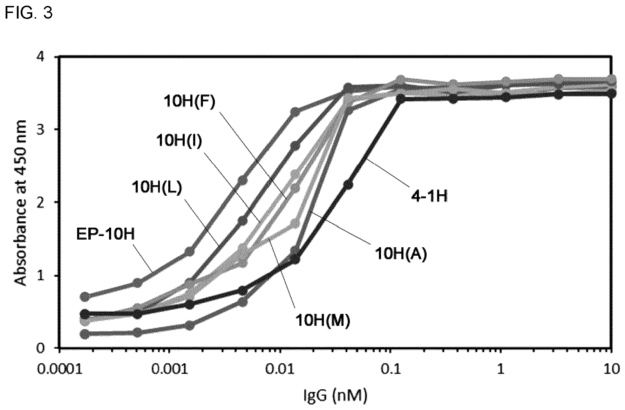

As an ELISA analysis result, the antibodies prepared by substituting the key site of binding ability with a hydrophobic amino acid showed higher affinity for CD55 than **4-1H** (FIG. 3). However, 10H(A), 10H(I), 10H(L), 10H(F) and 10H(M) showed lower affinity than EP-10H. This means that the substitution of G at the H100A site with V is the most effective for improving binding ability.

### Example 10: Therapeutic effect of EP-10H in CD55-overexpressed drugresistant cancer cells

### Example 10-1: Effect of co-administration of rituximab and EP-10H in Burkitt lymphoma BJAB cells

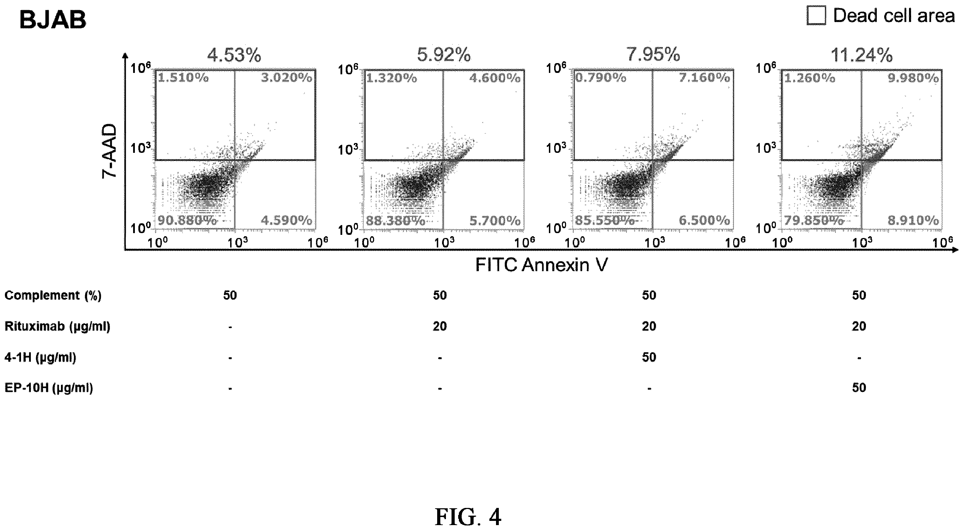

BJAB cells have complement resistance and this is due to the expression of CD55 (Golay, et al., Blood, 95 (12): 3900-8, 2000). For this reason, they show low response to rituximab the main therapeutic mechanism of which is CDC (complement-dependent cytotoxicity). In order to confirm the possibility of improving the therapeutic effect of rituximab for Burkitt lymphoma, which is known as malignant B cell lymphoma, the apoptotic ability of co-administration with EP-10H in Burkitt lymphoma BJAB cells was investigated. For investigation of the effect of co-administration, 5×10⁵ BJAB cells were cultured for 1 hour under the condition of 37 °C and 5% CO₂ after treating with 100 µL of RPMI supplemented with 50% complement human serum (Sigma, USA), containing 50 µg/mL of 4-1H or EP-10H and 20 µg/mL of rituximab. Then, CDC was observed by identifying apoptotic cells with Attune NxT (ThermoFisher Scientific, USA), which is a FACS analysis equipment, using an FITC annexin V apoptosis detection kit with 7-AAD (BioLegend, USA) (FIG. 4). As previously reported, the BJAB cells showed low response to rituximab, but the effect was increased when anti-CD55 antibodies (4-1H and EP-10H) were administered together. The increase was higher for a combination of rituximab and EP-10H than a combination of rituximab and 4-1H. This result indicates that the expression of CD55 inhibits the CDC effect of rituximab, and the inhibited effect is restored when CD55 is inhibited using the antibodies. It was confirmed that this effect was more significant for EP-10H having higher affinity for CD55.

### Example 10-2a: Confirmation of expression of CD55 in rituximab-resistant Ramos-RR cells

Ramos cells are rituximab-responsive B-cell lymphoma cells. The expression of CD55 in rituximab-resistant Ramos-RR cells prepared using Ramos cells was investigated as follows.

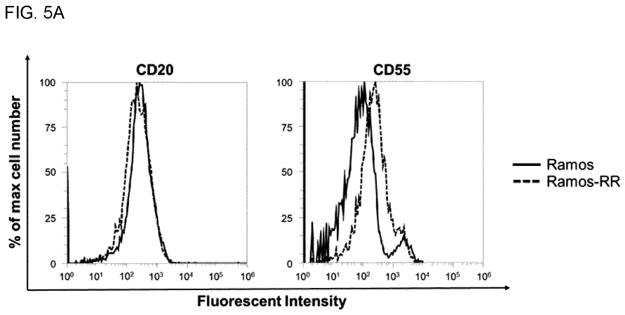

5×10⁵ Ramos or Ramos-RR cells were suspended in PBS containing or not containing 10 µg/mL of the EP-10H monoclonal antibody and cultured at 4 °C for 1 hour. The culture was centrifuged at 4,000 rpm for 5 minutes and centrifuged again at 4,000 rpm for 5 minutes after washing with 100 µL of PBS. After treating the cells with goat anti-human IgG antibody diluted to 1:100 using PBS and Alexa Fluor 488 (ThermoFisher Scientific, USA), they were incubated at 4 °C for 30 minutes in the shade. The fluorescence-stained cells were washed with PBS, suspended in 400 µL of PBS and then analyzed using Attune NxT (ThermoFisher Scientific, USA), which is a FACS analysis equipment. The result is shown in FIG. 5a.

It was confirmed that the expression of CD55 was increased in the Ramos-RR cells as compared to the Ramos cells. This indicates that CD55 is involved in the resistance to rituximab.

### Example 10-2b: Effect of co-administration of rituximab and EP-10H in rituximab-resistant Ramos-RR cells

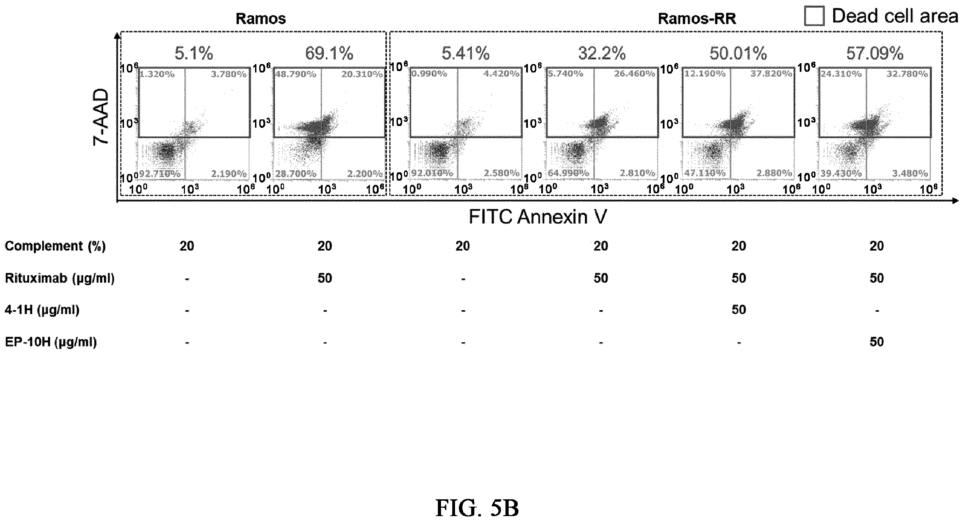

The apoptotic ability of co-administration of rituximab and EP-10H in Ramos-RR cells, which exhibit resistance to rituximab due to overexpression of CD55, was investigated according to the method of Example 10-1 (FIG. 5b). Whereas rituximab induced apoptosis in the Ramos cells, the induction of apoptosis in the Ramos-RR cells was lower than in the Ramos cells. However, the co-administration of EP-10H and 4-1H increased apoptosis in the Ramos cells and, as in the BJAB cells, the co-administration with EP-10H more significantly increased the apoptosis by rituximab as compared to the co-administration with 4-1H. This result shows that EP-10H can exhibit therapeutic effect in B-cell lymphoma, not only in primary cancers (BJAB cells are primary cancer cells) but also in carcinomas that acquired resistance to existing therapies.

### Example 10-3a: Expression of CD55 in imatinib-resistant K562-IR cells

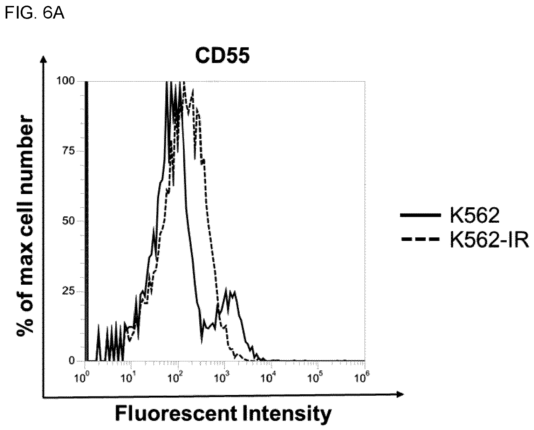

K562 cells are leukemia cells having responsiveness to imatinib, which is a tyrosine kinase inhibitor. The expression of CD55 in imatinib-resistant K562-IR cells prepared using K562 cells was investigated in the same manner as in Example 10-2a. As a result, it was confirmed that the expression of CD55 was increased in K562-IR cells as compared to K562 cells (FIG. 6a). This means that CD55 is involved in the resistance to imatinib.

### Example 10-3b: Effect of co-administration of imatinib and EP-10H in K562-IR cells

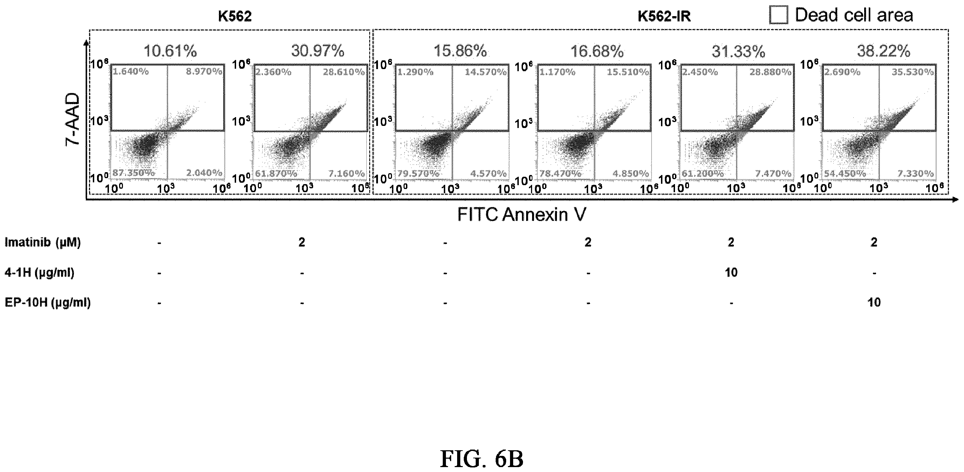

K562 cells and K562-IR cells, which exhibit resistance to imatinib due to overexpression of CD55, were seeded onto a 12-well cell culture plate at 2×10⁵ cells per well. Immediately thereafter, the cells were treated with 2 µM imatinib or with 2 µM imatinib and 10 µg/mL 4-1H or EP-10H diluted with 500 µL of RPMI, and then cultured under the condition of 37 °C and 5% CO₂ for 24 hours. Then, apoptotic cells were analyzed with the FACS analysis equipment Attune NxT using 7-AAD and an FITC annexin V apoptosis detection kit (FIG. 6b).

Imatinib induced apoptosis in the K562 cells but did not show the effect in the K562-IR cells. But, apoptosis was induced in the K562-IR cells when EP-10H or 4-1H was co-administered. In addition, the co-administration of EP-10H and imatinib exhibited higher apoptotic effect than the co-administration of 4-1H and imatinib. This result, along with the result of Example 10-3a, demonstrates that CD55 is the cause of resistance to imatinib, which is a tyrosine kinase inhibitor, and means that the inhibition of CD55 using EP-10H is an effective strategy to overcome the resistance to imatinib. Since imatinib is a long-term medication used for treatment of leukemia, a strategy to overcome drug resistance that can be induced by long-term medication is urgently needed. Therefore, the strategy for overcoming resistance to imatinib using EP-10H is expected to be very effective for treatment of leukemia.

### Example 10-4a: Expression of CD55 in gefitinib-resistant PC9-GR cells

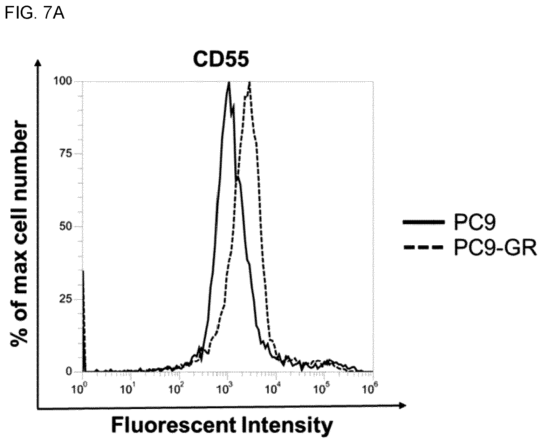

PC9 cells are non-small-cell lung cancer (NSCLC) cells having responsiveness to gefitinib, which is a tyrosine kinase inhibitor. As a result of investigating CD55 expression in gefitinib-resistant PC9-GR cells prepared using the PC9 cells according to the method of Example 10-2a, it was confirmed that the expression of CD55 was increased in the PC9-GR cells as compared to the PC9 cells (FIG. 7a). This suggests that CD55 is involved in the resistance to gefitinib.

### Example 10-4b: Effect of co-administration of gefitinib and EP-10H in PC9-GR cells

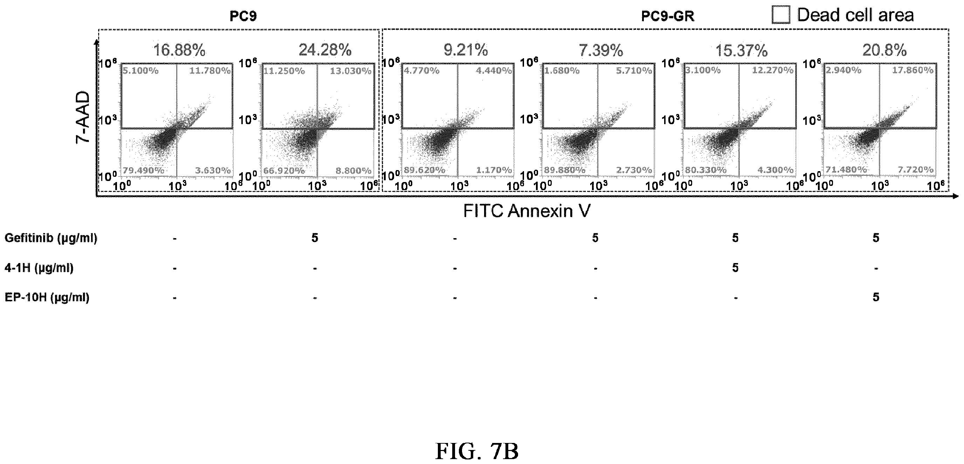

PC9 NSCLC cells and PC9-GR cells, which exhibit resistance to gefitinib due to overexpression of CD55, were seeded onto a 12-well cell culture plate at 1×10⁵ cells per well and cultured under the condition of 37 °C and 5% CO₂. 24 hours later, after treating with 5 µg/mL gefitinib or 5 µg/mL gefitinib and 5 µg/mL 4-1H or EP-10H diluted with 500 µL of RPMI, the cells were cultured again for 24 hours under the condition of 37 °C and 5% CO₂. Then, apoptotic cells were analyzed with the FACS analysis equipment Attune NxT using 7-AAD and an FITC annexin V apoptosis detection kit (FIG. 7b).

Gefitinib induced apoptosis in the PC9 NSCLC cells but did not show the effect in the PC9-GR cells. But, apoptosis was induced in the PC9-GR cells when EP-10H or 4-1H was co-administered with gefitinib. In addition, the co-administration of EP-10H and gefitinib exhibited higher apoptotic effect than the co-administration of 4-1H and gefitinib. This result, along with the result of Example 10-4a, demonstrates that CD55 is the cause of resistance to gefitinib, which is a tyrosine kinase inhibitor as a targeted anticancer drug, and means that the inhibition of CD55 using EP-10H is an effective strategy to overcome the resistance to gefitinib. Since gefitinib is a drug effective for NSCLC where there are not many therapeutic agents and it is difficult to select another drug, it is urgently needed to overcome the resistance to gefitinib. The strategy for overcoming resistance to imatinib using EP-10H is expected to be very effective for treatment of NSCLC.

### Example 11: Preparation of bispecific antibody using EP-10H and confirmation of efficacy

The effect of co-administration of EP-10H and various drugs was demonstrated in Example 10. Based on the result of co-administration with rituximab confirmed in Example 10-1 and Example 10-2, in order to compare the cancer cellkilling ability of co-administration of two antibodies targeting different antigens and a bispecific antibody capable of targeting two antigens at the same time, a CD20 x CD55 bispecific antibody was prepared using rituximab and EP-10H and the cancer cellkilling ability of the bispecific antibody was compared with the co-administration.

### Example 11-1. Bispecific antibody (CD20 x CD55) for expression in animal cells

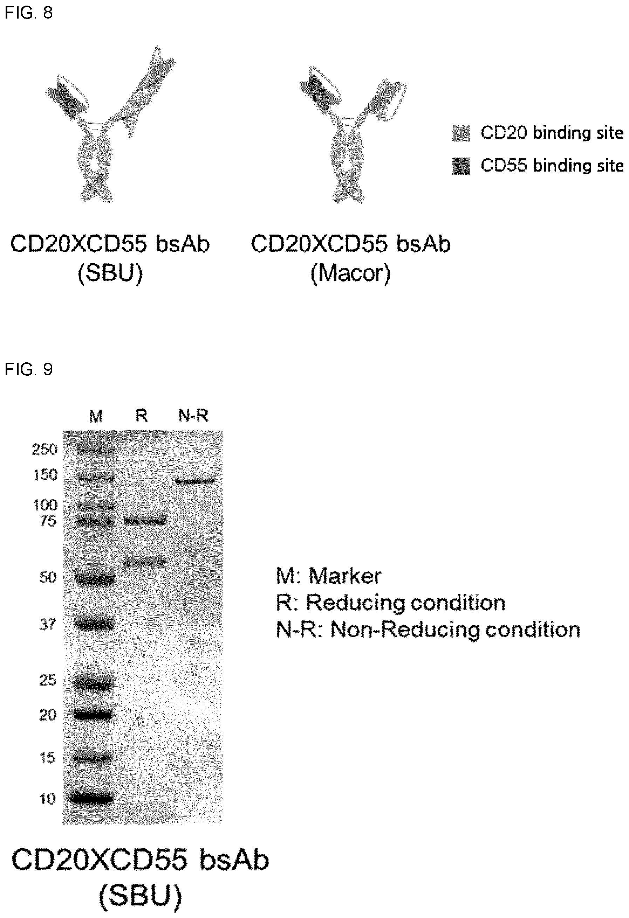

In the present disclosure, a novel bispecific antibody which is different from the previously bispecific antibody targeting CD20 and CD55 at the same time (Macor, et al., Leukemia, 29 (2): 406-14, 2014) was developed. For comparison of efficacy depending on the form of the bispecific antibody, the inventors of the present disclosure cloned a bispecific antibody targeting CD20 and CD55 at the same time using the genes of rituximab and EP-10H. The bispecific antibody prepared in the present disclosure was named SBU (specific bifunctional unit) (FIG. 8).

The CD20 x CD55 bispecific antibody (SBU) was prepared using the knobs-into-holes method to prevent unwanted pairing of heavy chains, and a linker consisting of Gly and Ser was used to prevent unwanted pairing of light chains. CD55-scFv-knob (V_{L}-linker20-V_{H}-C_{H}2-C_{H}3) and CD20-Fab-hole (V_{L}-C_{K}-linker40-V_{H}-C_{H}1-C_{H}2-C_{H}3) were cloned, respectively. For CD55-scFv-knob, PCR was conducted using the EP-10H prepared in Example 6 as a template and using the primer combinations described in Table 8. The amplified gene was treated with *Bss*Hll and *Xba*I enzymes (New England Biolabs, UK) and then ligated into a pMAZ vector, which is an animal cell expression vector treated with the same restriction enzymes. The ligated plasmid was transformed into DH5α competent cells (New England Biolabs, UK) by applying heat shock and the plasmid was obtained by culturing in large scale.

For preparation of CD20-Fab-hole (V_{L}-C_{K}-linker40-V_{H}-C_{H}1-C_{H}2-C_{H}3), PCR was conducted using the primer combinations described in Table 9 based on the sequence of rituximab published in Drug Data Bank. The amplified gene was treated with *Bss*Hll and *Xba*I enzymes and then ligated into a pMAZ vector, which is an animal cell expression vector treated with the same restriction enzymes. The ligated plasmid was transformed into DH5α competent cells (New England Biolabs, UK) by applying heat shock, and the plasmid was obtained by culturing in large scale and then sequenced (Table 10).

**[Table 8]**

| | Primers | Sequences | SEQ ID NO |
|---|---|---|---|
| V_{L} | Forward | CAG CGC GCA CTC CAG CTA CGA GCT GAC ACA GCC | 36 |
| | Reverse | GAC CAA GCT CAC CGT CTT GGG CGG AGG CGG GAG TGG TGG TGG CGG TAG CGG TGG AGG AGG C | 37 |
| V_{H} | Forward | GTG GAG GAG GCA GTG GAT CTG GCG GCT CTG AGG TCC AGC TGG TCG | 38 |
| | Reverse | GGC ACA ACC GTG ACC GTG TCC AGC GGC GGC TCA GAC AAA ACT CAC AC | 39 |
| C_{H} | Forward | CGT GTC CAG CGG CGG CTC AGA CAA AAC TCA CAC | 40 |
| | | ATG C | |
| | Reverse | CTC TCC CTG TCC CCG GGT AAA TGA CTA GAA CTA | 41 |

| | | | |
|---|---|---|---|
| Primers used in cloning of CD55-scFv-knob | | | |

**[Table 9]**

| | Primers | Sequences | SEQ ID NO |
|---|---|---|---|
| V_{L}-C_{K} | Forward | CGC AGC GAG CGC GCA CTC CCA GAT TGT CCT GTC TCA GTC TCC TGC | 42 |
| | Reverse | CGG CCG CCG TGC GAG ATC TTT TGA TTT CCA GTT TAG TTC CGC CG | 43 |
| V_{H} | Forward | CAA GTC CAA CTG CAA CAA CCG GG | 44 |
| | Reverse | GGA AGA CCG ATG GGC CCT TGA AGC TAG CGG CGG AAA CCG TTG TGC CAG AG | 45 |
| C_{H} | Forward | GCA AGC TTC AAG GGC | 46 |
| | Reverse | CTC TCC CTG TCC CCG GGT AAA TGA CTA GAA CTA | 41 |

| | | | |
|---|---|---|---|
| Primers used in cloning of CD20-Fab-hole | | | |

**[Table 10]**

| | | |
|---|---|---|
| CD55-scFv-knob | SYELTQPPSVSVSPGQTASITCSGGGGSYGWYQQKPGQS PVTVIYWNDKRPSGIPERFSGSKSGNTATLTISGTQAMDEA | SEQ ID NO 47 |
| | DYYCGGWDSSTYAIFGGGTKLTVLGGGGSGGGGSGGGGS GSGGSEVQLVESGGGLVQPGGSLRLSCAASGFTFSDRGM AWVRQAPGKGLEWVSGISSSGRYTYYAPAVKGRATISRDN SKNTLYLQMNSLRAEDTAVYYCAKNAVAGGWHAAYIDAWG QGTTVTVSSGGSDKTHTCPPCPAPELLGGPSVFLFPPKPKD TLMISRTPEVTCVVVDVSHEDPEVKFNWYVDGVEVHNAKTK PREEQYNSTYRVVSVLTVLHQDWLNGKEYKCKVSNKALPA PIEKTISKAKGQPREPQVYTLPPSRDELTKNQVSLWCLVKG FYPSDIAVEWESNGQPENNYKTTPPVLDSDGSFFLYSKLTV DKSRWQQGNVFSCSVMHEALHNHYTQKSLSLSPGK | |
| CD20-Fab-hole | QIVLSQSPAILSASPGEKVTMTCRASSSVSYIHWFQQKPGS SPKPWIYATSNLASGVPVRFSGSGSGTSYSLTISRVEAEDA ATYYCQQWTSNPPTFGGGTKLEIKRSRTAAAPSVFIFPPSD EQLKSGTASVVCLLNNFYPREAKVQWKVDNALQSGNSQES VTEQDSKDSTYSLSSTLTLSKADYEKHKVYACEVTHQGLSS PVTKSFNRGECGGGGSGGGGSGGGGSGSGGSGGGGSG GGGSGGGGSGSGSSQVQLQQPGAELVKPGASVKMSCKA SGYTFTSYNMHWVKQTPGRGLEWIGAIYPGNGDTSYNQKF KGKATLTADKSSSTAYMQLSSLTSEDSAVYYCARSTYYGG DWYFNVWGAGTTVTVSAASFKGPSVFPLAPSSKSTSGGTA ALGCLVKDYFPEPVTVSWNSGALTSGVHTFPAVLQSSGLYS LSSVVTVPSSSLGTQTYICNVNHKPSNTKVDKKVEPKSCDK THTCPPCPAPELLGGPSVFLFPPKPKDTLMISRTPEVTCVVV DVSHEDPEVKFNWYVDGVEVHNAKTKPREEQYNSTYRVVS | SEQ ID NO 48 |
| | VLTVLHQDWLNGKEYKCKVSNKALPAPIEKTISKAKGQPRE PQVYTLPPSRDELTKNQVSLSCAVKGFYPSDIAVEWESNG QPENNYKTTPPVLDSDGSFFLVSKLTVDKSRWQQGNVFSC SVMHEALHNHYTQKSLSLSPGK | |

| | | |
|---|---|---|
| Amino acid sequences of variable regions of CD55-scFv-knob and CD20-Fab-hole | | |

### Example 11-2. Expression in animal cells and purification

The DNA produced in Example 11-1 was expressed in the same manner as in Example 2. The CD20 x CD55 bispecific antibody (SBU) was transduced into HEK293F cells (Invitrogen, USA) using the plasmids of SEQ ID NOS 47 and 48, polyethyleneimine (PEI) (Polysciences, USA) and 150 mM NaCl and the cells were cultured for 7 days in a Freestyle 293 expression medium (Invitrogen, USA) under the condition of 37 °C, 8% CO₂ and 55% humidity. Although a bispecific antibody similar to SBU was reported (Moore, et al., A robust heterodimeric Fc platform engineered for efficient development of bispecific antibodies of multiple formats. Methods, 154: 38-50), three DNAs are required for expression for the bispecific antibody disclosed in the document. Unlike the existing bispecific antibody preparation method requiring three DNAs, the SBU of the present disclosure can be prepared with two DNAs because a linker consisting of Gly and Ser is used to prevent unwanted pairing of light chains. The culture of the expressed cells was centrifuged at 4,000 rpm or 10 minutes and the supernatant was filtered through a 0.22-µm filter. The filtered supernatant was induced to bind to 1 mL of KappaSelect resin (GE Healthcare, USA) at 4 °C. The resin was washed with a 10 cv (column volume) of 20 PBS and the bound antibody was eluted using a 100 mM glycine-HCl (pH 2.7) solution. After neutralizing with 1 M Tris-HCl (pH 9.0) and exchanging buffer with pH 7.2-7.4 PBS, the size and purity of the light chain and heavy chain of the purified antibody were identified finally by SDS-PAGE. As a result, a molecular weight consistent with the theoretical value and high purity were confirmed (FIG. 9). The SBU bispecific antibody was in hetero form with Fab as a CD20-binding site and scFv as a CD55-binding site. Through purification with protein L using the C kappa region of Fab, the desired heterodimer can be obtained selectively while preventing the formation of knob-knob homodimers or knob monomer known to be the most likely to be produced during purification of a bispecific antibody (Giese, et al., Biotechnol Prog, 34 (2): 397-404).

### Example 11-3. Confirmation of binding ability of bispecific antibody through ELISA

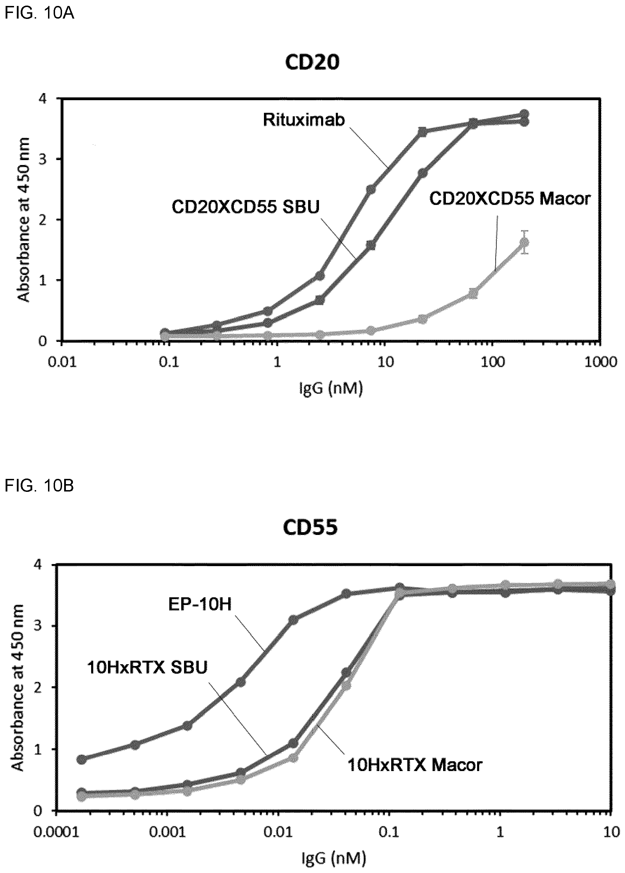

The affinity of the CD20 x CD55 bispecific antibody prepared in Example 11-2 for the antigens CD20 and CD55 was investigated by indirect ELISA. The indirect ELISA was conducted according to the method of Example 3. The bispecific antibody exhibited superior binding ability for both CD20 and CD55 (FIGS. 10a and 10b). In particular, the CD20 x CD55 bispecific antibody (SBU) showed higher affinity for CD20 than the CD20 x CD55 bispecific antibody reported by Macor and similar affinity for CD55 as the CD20 x CD55 bispecific antibody reported by Macor. It is expected that the CD20 x CD55 bispecific antibody (SBU) exhibiting higher affinity for CD20 will be more effective as a therapeutic agent for blood cancer wherein CD20 is overexpressed.

### Example 11-4. Apoptotic ability of CD20 x CD55 bispecific antibody in BJAB cells

The apoptotic ability of the CD20 x CD55 bispecific antibody was investigated by the method of Example 10-1. 5×10⁵ BJAB cells were treated with 20 µg/mL rituximab, 20 µg/mL EP-10H and rituximab or 20 µg/mL CD20 x CD55 bispecific antibody (SBU) diluted with 100 µL of RPMI containing 20% complement human serum (Sigma, USA). The other procedures were the same as in Example 10-1.

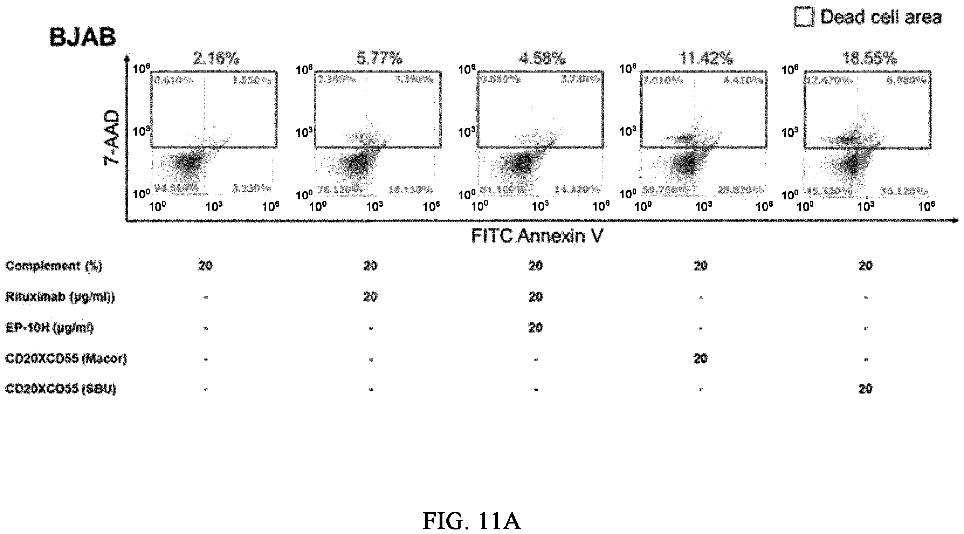

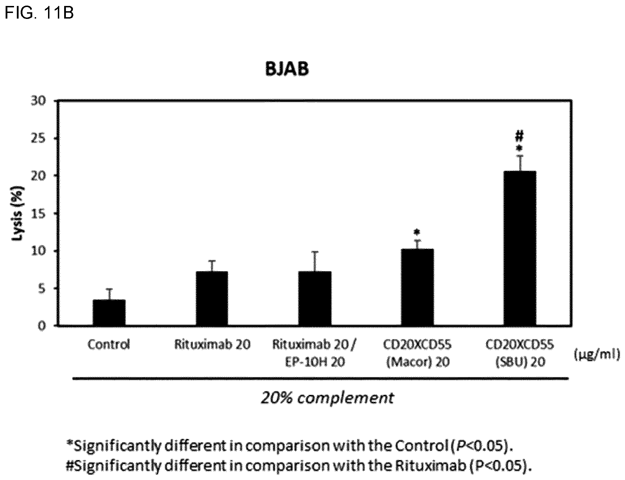

As a result, the CD20 x CD55 bispecific antibody showed significant difference in apoptotic ability in the BJAB cells exhibiting low responsiveness to rituximab as compared to the control group treated only with complement human serum. In particular, the SBU showed significant difference in the apoptotic ability as compared to rituximab (FIGS. 11a and 11b). This result suggests that one bispecific antibody capable of targeting two antigens at the same time is more effective in inducing CDC in cancer cells as compared to co-administration of two antibodies targeting different antigens, and the CD20 x CD55 bispecific antibody (SBU) of the present disclosure is more suitable for inducing CDC in cancer cells than the Macor's bispecific antibody. In addition, the CD20 x CD55 bispecific antibody (SBU) can be an effective therapeutic agent for Burkitt lymphoma which is known as malignant B-cell lymphoma.

### Example 11-5. Apoptotic ability of CD20 x CD55 bispecific antibody in Ramos cells

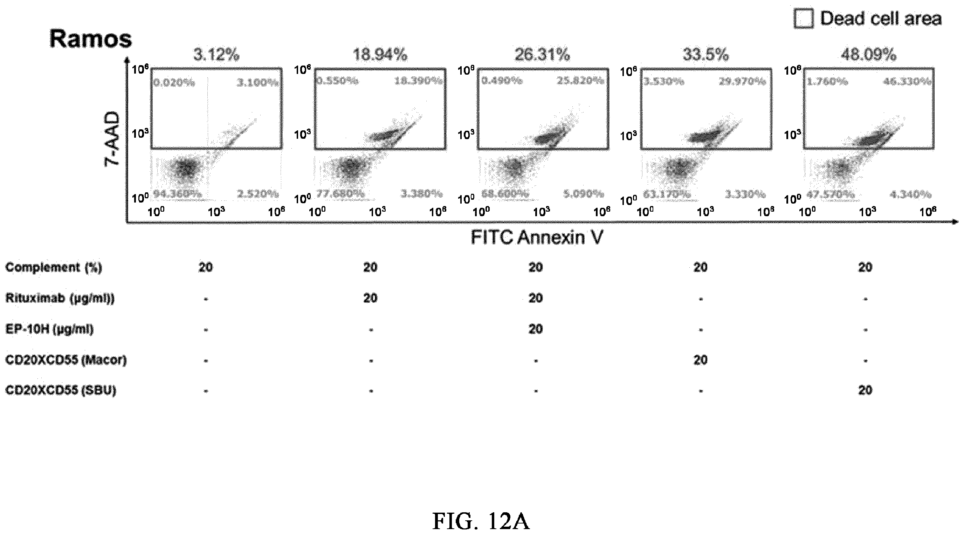

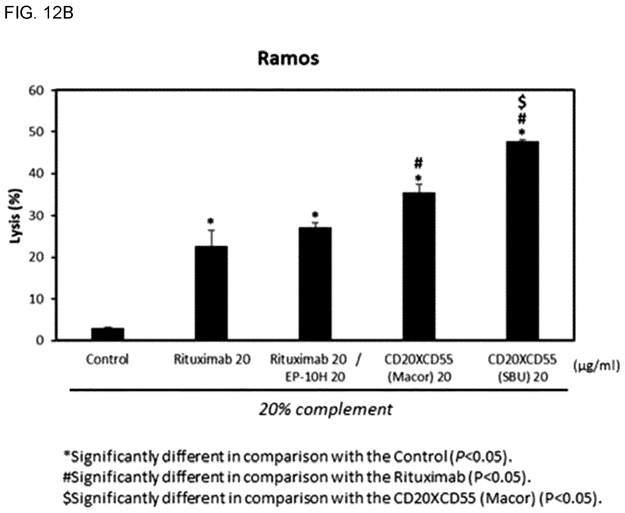

The apoptotic ability of the CD20 x CD55 bispecific antibody in Ramos cells was investigated by the method of Example 11-4 (FIGS. 12a and 12b). As a result, the CD20 x CD55 bispecific antibody showed significant difference in apoptotic ability in the Ramos cells exhibiting low responsiveness to rituximab as compared to rituximab. In addition, the SBU of the present disclosure showed better apoptotic ability than the Macor's bispecific antibody. This result suggests that the CD20 x CD55 bispecific antibody can exhibit more effective anticancer effect in rituximab-responsive carcinomas than rituximab, and the CD20 x CD55 bispecific antibody (SBU) can be an effective therapeutic agent for blood cancer wherein CD20 is expressed.

### Example 11-6. Apoptotic ability of CD20 x CD55 bispecific antibody in Ramos-RR cells

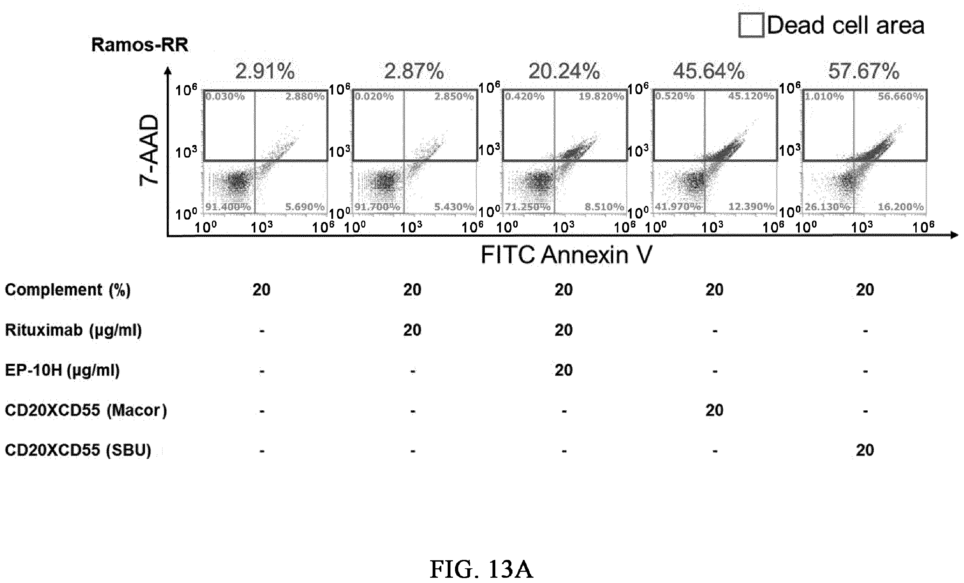

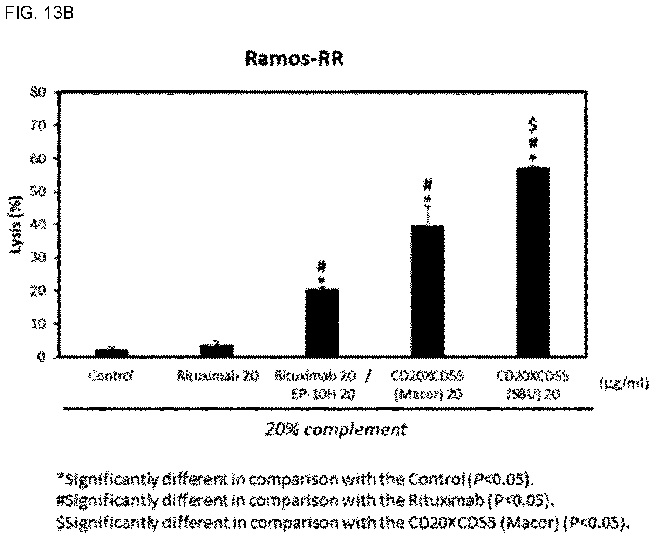

The apoptotic ability of the CD20 x CD55 bispecific antibody in Ramos-RR cells was investigated by the method of Example 11-4 (FIGS. 13a and 13b). As a result, the CD20 x CD55 bispecific antibody showed significant difference in apoptotic ability in the Ramos-RR cells exhibiting no responsiveness to rituximab as compared to rituximab. Consistent with the result for the BJAB cells, the SBU of the present disclosure showed remarkably superior apoptotic ability than the Macor's bispecific antibody. This result suggests that the CD20 x CD55 bispecific antibody (SBU) can be used as an effective therapeutic agent for CD20-expressed blood cancer showing resistance to rituximab.

### Example 11-7. Activation of CDC by CD20 x CD55 bispecific antibody (SBU)

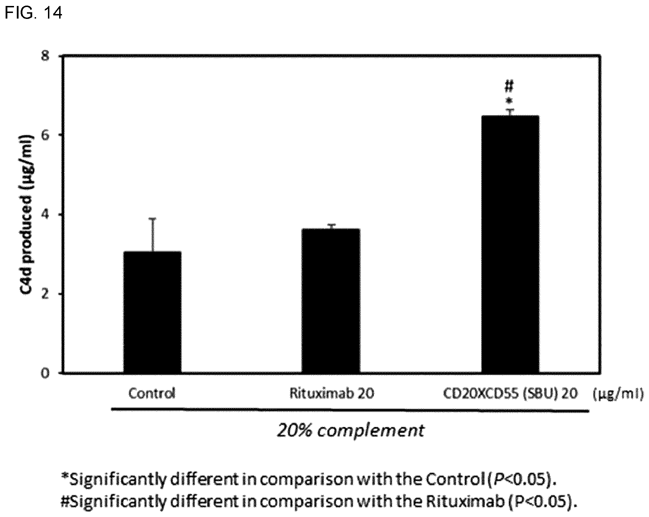

The supernatant treated with rituximab and the CD20 x CD55 bispecific antibody (SBU) in Example 11-5 was taken before cytometry of the Ramos-RR cell pellets and subjected to CDC activation analysis (FIG. 14). The CDC mechanism is mediated by binding of C1q to an antibody, and the activation of CDC can be analyzed based on the production of C4d, which is a byproduct of C1q. When analyzed using an ELISA kit (Quidel, USA) capable of identifying C4d production in the supernatant, more C4d was produced in the supernatant treated with the CD20 x CD55 bispecific antibody (SBU) as compared to the supernatant treated with rituximab, and the difference was significant. This result clearly shows that the superior apoptotic ability of the SBU CD20 x CD55 bispecific antibody is due to the mechanism of CDC activation.

While the specific exemplary embodiments of the present disclosure have been described in detail above, it will be obvious to those having ordinary knowledge in the art that the specific exemplary embodiments are merely preferred exemplary embodiments and the scope of the present disclosure is not limited by them. Accordingly, the substantial scope of the present disclosure should be defined by the appended claims and their equivalents.

## Claims

1. An antibody binding specifically to CD55 or an antigen-binding fragment thereof, comprising a heavy chain variable region comprising an HCDR1 region having an amino acid sequence represented by the general formula 1; an HCDR2 region having an amino acid sequence of SEQ ID NO 1; and an HCDR3 region having an amino acid sequence represented by the general formula 2:
**General formula 1** D-R-G-M-X₁
**General formula 2** N-A-X₂-A-G-G-W-H-A-A-Y-I-D-A
wherein X₁ is Ala or Val, and X₂ is selected from a group consisting of Val, Ala, Ile, Leu, Phe and Met.

2. The antibody or antigen-binding fragment thereof according to claim 1, wherein X₁ is Ala.

3. The antibody or antigen-binding fragment thereof according to claim 1, wherein X₂ is Val.

4. The antibody or antigen-binding fragment thereof according to claim 1, wherein the antibody or antigen-binding fragment thereof further comprises a light chain variable region comprising an LCDR1 region having an amino acid sequence of SEQ ID NO 2; LCDR2 region having an amino acid sequence represented by the general formula 3; and an LCDR3 region having an amino acid sequence of SEQ ID NO 3:
**General formula 3** W-N-X₃-K-R-P-S
wherein X₃ is Asn or Asp.

5. The antibody or antigen-binding fragment thereof according to claim 4, wherein X₃ is Asp.

6. An antibody binding specifically to CD55 or an antigen-binding fragment thereof, comprising:
a) a light chain variable region of SEQ ID NO 4; or a light chain variable region comprising one or more substitution selected from a group consisting of substitution of Y (Tyr) of the 32nd amino acid sequence in the light chain variable region of SEQ ID NO 4 with F (Phe) and substitution of D (Asp) of the 48th amino acid sequence with N (Asn), and
b) a heavy chain variable region of SEQ ID NO 5; or a heavy chain variable region comprising one or more substitution selected from a group consisting of substitution of V (Val) of the 5th amino acid sequence in the heavy chain variable region of SEQ ID NO 5 with A (Ala), substitution of A (Ala) of the 23rd amino acid sequence with V (Val), substitution of S (Ser) of the 25th amino acid sequence with R (Arg), substitution of A (Ala) of the 35th amino acid sequence with V (Val), substitution of A (Ala) of the 40th amino acid sequence with S (Ser), substitution of A (Ala) of the 68th amino acid sequence with T (Thr), substitution of S (Ser) of the 85th amino acid sequence with N (Asn) and substitution of G (Gly) of the 101st amino acid sequence with V (Val), L (Leu), I (Ile), F (Phe), M (Met) or A (Ala).

7. The antibody or antigen-binding fragment thereof according to claim 1 or 6, wherein the antigen-binding fragment is scFv, an Fab fragment, an F(ab') fragment, an F(ab')₂ fragment or an Fv fragment.

8. A nucleic acid molecule encoding the antibody or antigen-binding fragment thereof according to any of claims 1 to 6.

9. A pharmaceutical composition for preventing or treating cancer, comprising the antibody or antigen-binding fragment thereof according to any of claims 1 to 6 as an active ingredient.

10. The composition according to claim 9, wherein the composition further comprises one or more pharmaceutical ingredient selected from a group consisting of an anti-CD20 antibody and a tyrosine kinase inhibitor.

11. The composition according to claim 10, wherein the anti-CD20 antibody is rituximab.

12. The composition according to claim 10, wherein the tyrosine kinase inhibitor is one or more inhibitor selected from a group consisting of imatinib and gefitinib.

13. The composition according to claim 9, wherein the cancer is CD55-positive cancer.

14. A composition for diagnosing cancer, comprising the antibody or antigen-binding fragment thereof according to any of claims 1 to 6 as an active ingredient.

15. The composition according to claim 14, wherein the cancer is CD55-positive cancer.

16. A bispecific antibody comprising the antigen-binding fragment of the antibody binding specifically to CD55 according to any of claim 1 to 6 and an antigen-binding fragment of an antibody binding specifically to CD20, or a functional fragment thereof.

17. The bispecific antibody or functional fragment thereof according to claim 16, wherein the antibody binding specifically to CD20 is rituximab.

18. The bispecific antibody or functional fragment thereof according to claim 16, wherein the antigen-binding fragment of the antibody binding specifically to CD55 comprises scFv.

19. The bispecific antibody or functional fragment thereof according to claim 16, wherein the antigen-binding fragment of the antibody binding specifically to CD55 is V_{L}-linker-V_{H}-C_{H}2-C_{H}3.

20. The bispecific antibody or functional fragment thereof according to claim 16, wherein the antigen-binding fragment of the antibody binding specifically to CD20 comprises Fab.

21. The bispecific antibody or functional fragment thereof according to claim 16, wherein the antigen-binding fragment of the antibody binding specifically to CD20 is V_{L}-C_{K}-linker-V_{H}-C_{H}1-C_{H}2-C_{H}3.
